(19) Europäisches Patentamt
European Patent Office
Office européen des brevets

(11) EP 1 588 760 A1

# (12) EUROPEAN PATENT APPLICATION

(43) Date of publication:
**26.10.2005 Bulletin 2005/43**

(51) Int Cl.[7]: **B01J 13/00**, A61K 7/50, A61K 7/16, A61K 7/46

(21) Application number: **05252299.2**

(22) Date of filing: **13.04.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **13.04.2004 US 823492**

(71) Applicant: **INTERNATIONAL FLAVORS & FRAGRANCES INC.**
**New York New York 10019 (US)**

(72) Inventors:
- **Brooks, Clint Dee Winton**
  **Sea Bright, NJ 07760 (US)**
- **Popplewell, Lewis Michael**
  **Morganville, NJ 07751 (US)**
- **Semoff, Steven**
  **New City, NY 10956 (US)**
- **Lindauer, Jerome I.**
  **Hillsdale, NJ 07642 (US)**
- **Lee, Kaiping**
  **Morganville, NJ 07751 (US)**
- **Zhen, Yueqian**
  **Tredyffrin, PA 19301 (US)**
- **Prol, Melanie Beth**
  **Brick, NJ 08724 (US)**
- **Pluyter, Johan G. L.**
  **Middletown, NJ 07748 (US)**

(74) Representative: **Mercer, Christopher Paul et al**
**Carpmaels & Ransford,**
**43-45 Bloomsbury Square**
**London WC1A 2RA (GB)**

## (54) Skin and hair treatment composition and process for using same resulting in controllably-releasable fragrance and/or malodour counteractant evolution

(57) Described is a human epidermal and/or human hair treatment composition of (i) fragrance and/or malodour counteractant and (ii) fragrance- and/or malodour counteractant-compatible solvent-containing microcapsules provided in a suspension gel or emulsion. Also described is a method for delivery of fragrance and beneficial agents to the human epidermal surface area region treatment and/or human hair treatment wherein the microcapsules remain on the human epidermis and/or on the hair follicles for a substantial period of time subsequent to their application and the microcapsules are ruptured over a period of time, thereby effecting both an initial delivery of fragrance and beneficial agent subsequent to rubbing, and, in addition, a controlled release of fragrance and beneficial agents into to the treated human epidermis and/or hair follicles.

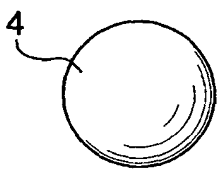


FIG. 1

EP 1 588 760 A1

## Description

### FIELD OF THE INVENTION

Aqueous suspensions of rupturable, fragrance- and/or malodour counteractant-containing microcapsules and process for using same in the treatment of regions of the human epidermis and/or human hair.

### BACKGROUND OF THE INVENTION

The need for targeted delivery of fragrances and malodour counteractants to specific regions of the human epidermis and to specific human hair follicle groups and to the environment proximate thereto with at least a modicum of permanency and, in addition, the need for simultaneously providing an initial 'burst' of fragrance and/or malodour counteractant is well-recognized. Thus, application of colognes, after-shave lotions, after-bath preparations and splash lotions containing fragrance-containing microcapsules, including those having walls fabricated from urea-formaldehyde polymers, and non-confined fragrances to skin is disclosed in U.S. Patent 4,428,869. In addition, hair conditioners and body washes containing fragrances which are encapsulated in capsules having walls fabricated from polymers which are reaction products of melamine-formaldehyde precondensates with ethylene/maleic anhydride copolymers are disclosed in PCT published patent application WO 02/074430 A1. However, nothing set forth in the prior art either describes or suggests a technique for highly substantive deposition of effectively-rupturable malodour suppressant and/or fragrance emitting microcapsules onto specific regions of the human epidermis or onto groups of human hair follicles wherein the resulting emitted fragrance activity and/or malodour counteractant activityt hat is continuously intense and long-lasting and where the resulting substantive aroma is aesthetically pleasing over the long period of time during which it is effective.

### SUMMARY OF THE INVENTION

Our invention provides a composition for effecting the direct deposition of effectively-malodour suppressant and/or fragrance emitting microcapsules onto specific regions of the human epidermis or onto groups of human hair follicles wherein the resulting emitted fragrance activity and/or malodour counteractant activity is continuously intense and long-lasting and where the resulting substantive aroma is aesthetically pleasing over the long period of time during which it is effective.

More specifically, our invention provides a human epidermal and/or human hair treatment composition of fragrance and/or malodour counteractant and fragrance-compatible and/or malodour counteractant-compatible hydrophobic solvent-containing microcapsules suspended in a solution, an aqueous gel or emulsion and the utilization of such compositions for human epidermal surface area region treatment and/or human hair treatment wherein the microcapsules remain on the human epidermis and/or on the hair follicles for a substantial period of time subsequent to their application and the microcapsules are over a period of time, thereby effecting both an initial fragrance and/or malodour counteractant burst immediately subsequent to rubbing, and, in addition, a long-term controlled release of fragrance and/or malodour counteractant into the environment proximate the treated human epidermis and/or hair follicles.

Optionally, the human epidermal and/or hair treatment composition can also contain non-confined fragrance thereby effecting an initial fragrance burst immediately subsequent to application in addition to the fragrance burst immediately subsequent to rubbing and to the long-term controlled release of fragrance and/or malodour counteractant into the environment proximate the treated human epidermis and/or hair follicles.

Still more specifically, our invention is directed to a human epidermal and/or hair treatment composition comprising in intimate admixture (i) a plurality of microcapsules each of which (a) has an outside diameter in the range of from about 0.01 to about 1000 microns; (b) has a microcapsule wall having a thickness in the range of from about 0.001 to about 100 microns; (c) has a microcapsule wall preferably composed of a substituted or un-substituted acrylamide-acrylic acid co-polymer cross-linked with a melamine-formaldehyde pre-condensate; and (d) has a liquid phase core further comprising a fragrance and/or a malodour counteractant composition, the majority of which components by weight have a C $\log_{10}P$ of from about 1.5 to about 8.0 and a hydrophobic solvent composition each of the components of which (A) is compatible with each of the components of said fragrance and/or malodour counteractant composition and (B) has a C $\log_{10}P'$ greater than about 2.0, preferably greater than 3.3 and (ii) an aqueous human epidermal skin treatment and/or hair treatment base which maintains the microcapsules in suspension.

The aqueous human epidermal skin treatment and/or hair treatment base for maintaining in suspension the microcapsules may be, in the alternative, a gel base or an emulsion base. The concentration of fragrance components in the human epidermal and/or hair treatment composition of our invention is in the range of from about 0.1 to about 50.0% by weight of the composition. The microcapsules of the present invention can be used to deliver all of the

fragrance in the product or can be used with product bases in which there is free or non-encapsulated fragrance in the base.

The range of weight percent of fragrance composition components and/or malodour counteractant composition components in the microcapsules used in the practice of our invention is from about 5 % to about 98 % by weight, preferably form about 10 to about 90 and most preferably from about 25 to about 75 weight percent of the filled microcapsule. Solvent is preferably included with the fragrance composition and / or malodor benefit agents. The range of weight percent of solvent in the microcapsules is preferably form about 10 to about 90 and most preferably from about 25 to about 75 weight percent of the filled microcapsules.

The core materials in the microcapsules may be comprised wholly of hydrophobic solvent, or other non-fragrance benefit agent. These capsules can have several potential functions. First, they may serve to deliver benefit agent to the skin in a controllable manner. Second, solvent loaded capsules may absorb free fragrance delivered as part of the treatment base and then controllably release it. Third, solvent loaded capsules may absorb malodor compounds present on the hair or skin, thus reducing their headspace concentration and aroma.

Our invention is also directed to a process for treating a given region of the human epidermis and/or human hair, e.g., group of human hair follicles, comprising the step of applying to the given region of the human epidermis and/or hair a human epidermis and/or hair treating quantity of the above-mentioned composition over a human epidermis and/or hair treatment period of time.

## DETAILED DESCRIPTION OF THE INVENTION

The human epidermal and/or hair treatment composition of our invention comprises a suspension of (i) a plurality of microcapsules filled with a fragrance composition and/or a malodour counteractant composition in admixture with a compatible solvent in (ii) an aqueous gel base or an aqueous emulsion base.

### The Microcapsules

Encapsulation of fragrances is known in the art, see for example U.S. Patent Nos. 2,800,457, 3,870,542, 3,516,941, 3,415,758, 3,041,288, 5,112,688, 6,329,057, and 6,261,483. Another discussion of fragrance encapsulation is found in the Kirk-Othmer Encyclopedia.

Preferred encapsulating polymers include those formed from melamine-formaldehyde or urea-formaldehyde condensates, as well as similar types of aminoplasts. Additionally, capsules made via the simple or complex coacervation of gelatin are also preferred for use with the coating. Capsules having shell walls comprised of polyurethane, polyamide, polyolefin, polysaccaharide, protein, silicone, lipid, modified cellulose, gums, polyacrylate, polyphosphazines, polystyrene, and polyesters or combinations of these materials are also functional.

A representative process used for aminoplast encapsulation is disclosed in U.S. Patent No. 3,516,941 though it is recognized that many variations with regard to materials and process steps are possible. A representative process used for gelatin encapsulation is disclosed in U.S. Patent No, 2,800,457 though it is recognized that many variations with regard to materials and process steps are possible. Both of these processes are discussed in the context of fragrance encapsulation for use in consumer products in which the present invention is suitable include shampoos, hair conditioners and deodorants in U.S. Patent Nos. 4,145,184 and 5,112,688 respectively.

Well known materials such as solvents, surfactants, emulsifiers, and the like can be used in addition to the polymers described above to encapsulate the fragrance without departing from the scope of the present invention. It is understood that the term encapsulated is meant to mean that the fragrance material is substantially covered in its entirety. Encapsulation can provide pore vacancies or interstitial openings depending on the encapsulation techniques employed. More preferably the entire fragrance material and malodour counteractant product portion of the present invention is encapsulated.

Particles comprised of fragrance and a variety of polymeric and non-polymeric matrixing materials are also suitable for use. These may be composed of polymers such as polyethylene, fats, waxes, or a variety of other suitable materials. Essentially any capsule, particle, or dispersed droplet may be used that is reasonably stable in the application and release of fragrance or malodour counteractant at an appropriate time once deposited.

The microcapsule walls are preferably composed of an aminoplast resin, more specifically a substituted or un-substituted acrylic acid polymer or co-polymer cross-linked with a urea-formaldehyde pre-condensate or a melamine-formaldehyde pre-condensate. The microcapsule is formed by means of either (a) forming an aqueous dispersion of a non-cured aminoplast resin by reacting under acidic pH conditions a urea-formaldehyde pre-condensate or a melamine-formaldehyde pre-condensate with one or more substituted or un-substituted acrylic acid polymers or co-polymers; then coacervating the resulting non-cured aminoplast resin shell about the surface of a fragrance and/or malodour counteractant-solvent monophasic droplet under homogenization conditions such as using a homogenization apparatus as described in U.S. Patent 6,042,792 and illustrated in Figures 11A and 11B thereof; and then curing the

microcapsule shell wall at an elevated temperature, e.g. 50 - 85°C.

The urea-formaldehyde and melamine-formaldehyde pre-condensate microcapsule shell wall precursors are prepared by means of reacting urea or melamine with formaldehyde where the mole ratio of melamine or urea to formaldehyde is in the range of from about 10:1 to about 1:6, preferably from about 1:2 to about 1:5. For purposes of practicing our invention, the resulting material has a molecular weight in the range of from 156 to 3000. The resulting material may be used 'as-is' as a cross - linking agent for the aforementioned substituted or un-substituted acrylic acid polymer or copolymer or it may be further reacted with a $C_1$-$C_6$ alkanol, e.g., methanol, ethanol, 2-propanol, 3-propanol, 1-butanol, 1-pentanol or 1-hexanol, thereby forming a partial ether where the mole ratio of melamine or urea:formaldehyde:alkanol is in the range of 1:(0.1 - 6):(0.1-6). The resulting ether moiety-containing product may by used 'as-is' as a cross-linking agent for the aforementioned substituted or un-substituted acrylic acid polymer or copolymer, or it may be self-condensed to form dimers, trimers and/or tetramers which may also be used as cross-linking agents for the aforementioned substituted or un-substituted acrylic acid polymers or co-polymers. Methods for formation of such melamine-formaldehyde and urea-formaldehyde pre-condensates are set forth in U.S. Patent 3,516,846, U.S. Patent 6,261,483, and Lee et al. J. Microencapsulation, 2002, Vol. 19, No. 5, pp 559-569, "Microencapsulation of fragrant oil via in situ polymerization: effects of pH and melamine-formaldehyde molar ratio". Examples of urea-formaldehyde pre-condensates useful in the practice of our invention are URAC 180 and URAC 186, Cytec Technology Corp. Examples of melamine-formaldehyde pre-condensates useful in the practice of our invention are CYMEL U-60, CYMEL U-64 and CYMEL U-65 manufactured by Cytec Technology Corp.

The average outside diameter of the resulting microcapsule is in the range of from about 0.01 microns to about 1000 microns; preferably from about 0.05 microns to about 100 microns and more preferably from about 2.0 microns to about 20 microns. The average wall thickness of the resulting microcapsule is in the range of from about 0.001 microns to about 100 microns; preferably from about 0.005 microns to about 10 microns and more preferably from about 0.2 microns to about 2.0 microns. The microcapsules are friable or rupturable, particularly when dried which release the fragrance and other agents at the appropriate time to the user.

The content of the resulting microcapsule includes a fragrance composition a malodour counteractant composition and beneficial agent, preferably in combination with a compatible hydrophobic solvent. The term "compatible" is herein intended to mean essentially chemically non-reactive with every fragrance component, malodour counteractant and beneficial agent component and normally capable of forming a single liquid. In the practice of our invention, the range of weight percent of solvent/fragrance malodour counteractant and beneficial agent composition components contained in the microcapsules is from about 5% to about 98% by weight of the microcapsule, preferably from about 50% to about 97%, most preferably from about 91 % to 96%. Thus, the range of weight ratios of encapsulating polymer to solvent/fragrance malodour counteractant and beneficial agent components is preferably from about 1:25 to about 1:1; most preferably from about 1:10 to about 4:96. In addition, the range of weight percent of solvent in the microcapsule is preferably from about 10% to 80% by weight of the filled microcapsule. In a highly preferred ratio of weight of solvent: weight of encapsulated fragrance composition and/or encapsulated malodour counteractant composition is from about 2:1 to about 1:2, with the most preferred ratio being 1:1.

The compatible solvent, which is commonly a hydrophobic material, used in combination with the fragrance composition malodour counteractant composition and beneficial agent is preferably a mono-, di- or tri-$C_4$-$C_{26}$ saturated or unsaturated fatty acid glyceride, diethyl phthalate, dibutyl phthalate, diisodecyl adipate, a liquid polydimethyl siloxane, a liquid polydimethylcyclosiloxane, the methyl ester of soya fatty acid, a mixture of soya fatty acid methyl ester and isopropyl myristate with the weight ratio of soya fatty acid:isopropyl myristate being from 2:1 to 20:1 and a mineral oil compatible with each component of said fragrance composition and/or said malodour counteractant composition. More preferably, the solvent is a tri-$C_4$-$C_{26}$ saturated or unsaturated fatty acid glyceride. Most preferably, the solvent is the triglyceride ester of a mixture of caprylic acid and capric acid, commercially available as NEOBEE M-5 available from Stepan Chemical Company. The C $\log_{10}P'$ of the solvent is greater than 3.3, where P' is the n-octanol/water partition coefficient of the hydrophobic solvent is preferably greater than about 8 and most preferably greater than about 10.

The C $\log_{10}P$ of the components of the encapsulated fragrance composition and/or the encapsulated malodour counteractant composition is in the range of from about 1.5 to about 8, where P is the n-octanol/water partition coefficient of the fragrance component.

Another embodiment of the invention is the use of a vast preponderance, more than about 75 weight percent of fragrance materials with ClogP greater than 3.3, preferably greater than 4. In this embodiment of the invention greater than about 60 weight percent of the fragrance materials have a ClogP of greater than 3.3. In another highly preferred embodiment of the invention more than 80 weight percent of the fragrances have a ClogP value of greater than about 4.0.

Another embodiment is the use of significant levels of appropriate hydrophobic solvents in the fragrance core. Preferably greater than 30% of the core should consist of a hydrophobic solvent, and preferably that solvent should be selected from the group consisting of triglyceride oil, mono and diglycerides, mineral oil, silicone oil, polyalphaolefins,

fatty alcohols, diethyl phthalate, and isopropyl myristate.

A third embodiment involves the use of hydrophobic polymers in the core to reduce leaching. Preferably less than about 20% weight percent of polymer is used, and preferably that polymer is selected from the group consisting of ethyl cellulose, hydroxypropyl cellulose, ethylene vinyl acetate, polystyrene, and polyvinyl pyrrolidone and ester terminated polyamides or amide terminated polyamides.

The values of C $\log_{10}P$ of many functional product ingredients, for example fragrance ingredients contained in personal treatment compositions and/or cosmetic compositions is discussed in U.S. Patents 5,783,544 and 6,528,013. Furthermore, values of $\log_{10}P$ have been reported; for example, the Pomona92 database, available from Daylight Chemical Information Systems, Inc., Daylight CIS, Irvine, California. However, the $\log_{10}P$ values are most conveniently calculated by the "CLOGP" program, also available from Daylight CIS. This program also lists experimental $\log_{10}P$ values when they are available in the Pomona92 database. The "calculated $\log_{10}P$" (C $\log_{10}P$) is determined by the Hansch and Leo "fragment" approach based on the chemical structure of each functional product ingredient, and takes into account the numbers and types of atoms, the atom connectivity and the chemical bonding. The C $\log_{10}P$ values which are the most reliable and widely used estimates for this physicochemical property, are preferably used instead of the experimental $\log_{10}P$ values for the selection of functional ingredients, including perfume ingredients which are useful components in the microencapsulate-containing slurries of our invention.

Specific examples of preferred fragrance components useful in the aminoplast microencapsulates used in the composition and process of our invention, and the molecular weights and $Clog_{10}P$ values of each of said components are set forth in Table I as follows:

TABLE I

| **Fragance Component** | **$Clog_{10}P$ value** | **Molecular Weight** |
|---|---|---|
| amyl salicylate | 4.601 | 208.26 |
| benzyl salicylate | 4.383 | 228.25 |
| β-caryophyllene | 6.333 | 204.36 |
| ethyl undecylenate | 4.888 | 212.34 |
| geranyl anthranilate | 4.216 | 273.38 |
| α-irone | 3.820 | 206.33 |
| β-phenyl ethyl benzoate | 4.058 | 226.28 |
| α-santalol | 3.800 | 220.36 |
| amyl salicylate | 4.601 | 208.26 |
| β-caryophyllene | 6.333 | 204.36 |
| cedrol | 4.530 | 222.37 |
| cedryl acetate | 5.436 | 264.41 |
| cedryl formate | 5.070 | 238.37 |
| cyclohexyl salicylate | 5.265 | 220.29 |
| γ-dodecalactone | 4.359 | 198.31 |
| β-phenylethyl phenyl acetate | 3.767 | 240.31 |
| 5-acetyl-1,1,2,3,3,6-hexamethyl indane | 5.977 | 25 8.41 |
| cyclopentadecanolide | 6.246 | 240.39 |
| amyl cinnamic aldehyde | 4.324 | 202.30 |
| linalyl benzoate | 5.233 | 258.36 |

The performance of the capsules of the present invention may be improved through the use of a vast preponderance of high ClogP fragrance materials. In this embodiment of the invention greater than about 60 weight percent of the fragrance materials have a ClogP of greater than 3.3. In another highly preferred embodiment of the invention more than 80 weight percent of the fragrances have a ClogP value of greater than about 4.0. Use of fragrance materials

as described previously reduces the diffusion of fragrance through the capsule wall and into the base under specific time, temperature, and concentration conditions.

The higher ClogP materials are preferred, meaning that those materials with a ClogP value of 4.5 are preferred over those fragrance materials with a ClogP of 4; and those materials are preferred over the fragrance materials with a ClogP of 3.3.

The fragrance formulation of the present invention should have at least about 60 weight percent of materials with ClogP greater than 3.3, preferably greater than about 80 and more preferably greater than about 90 weight percent of materials with ClogP greater than 4.

Those with skill in the art appreciate that fragrance formulations are frequently complex mixtures of many fragrance ingredients. A perfumer commonly has several thousand fragrance chemicals to work from. Those with skill in the art appreciate that the present invention may contain a single ingredient, but it is much more likely that the present invention will comprise at least eight or more fragrance chemicals, more likely to contain twelve or more and often twenty or more fragrance chemicals. The present invention also contemplates the use of complex fragrance formulations containing fifty or more fragrance chemicals, seventy five or more or even a hundred or more fragrance chemicals in a fragrance formulation.

Preferred fragrance materials will have both high ClogP and high vapor pressure. Among those having these properties are:

Para cymene, Caphene, Mandarinal Firm, Vivaldie, Terpinene, Verdox, Fenchyl acetate, Cyclohexyl isovalerate, Manzanate, Myrcene, Herbavert, Isobutyl isobutyrate, Tetrahydrocitral, Ocimene and Caryophyllene.

Shampoo and hair conditioners that can employ the present invention include those products described in U. S. Patents 6,162,423, 5,968,286, 5,935,561, 5,932,203, 5,837,661, 5,776,443, 5,756,436, 5,661,118, 5,618,523, 5,275,755, 5,085,857, 4,673,568, 4,387,090 and 4,705,681.

In addition to the fragrance materials that are to be encapsulated in the present invention, the present invention also contemplates the incorporation of solvent materials. The solvent materials are hydrophobic materials that are preferably miscible in the fragrance materials used in the present invention. Suitable solvents are those having reasonable affinity for the fragrance chemicals and a ClogP greater than 3.3, preferably greater than 8 and most preferably greater that 10. Suitable materials include, but are not limited to triglyceride oil, mono and diglycerides, mineral oil, silicone oil, diethyl phthalate, polyalpa olefms, castor oil and isopropyl myristate. In a preferred embodiment the solvent materials are combined with fragrance materials that have high ClogP values as set forth above. It should be noted that selecting a solvent and fragrance with high affinity for each other will result in the most pronounced improvement in stability.

Appropriate solvents may be selected from the following non-limiting list:

- Mono-, di- and tri-esters, and mixtures thereof, of fatty acids and glycerine. The fatty acid chain can range from C4-C26. Also, the fatty acid chain can have any level of unsaturation. For instance capric/caprylic triglyceride known as Neobee M5 (Stepan Corporation). Other suitable examples are the Capmul series by Abitec Corporation. For instance, Capmul MCM.
- Isopropyl myristate
- Fatty acid esters of polyglycerol oligomers:

    R2CO-[OCH2-CH(OCOR1)-CH2O-]n, where R1 and R2 can be H or C4-26 aliphatic chains, or mixtures thereof, and n ranges between 2 - 50, preferably 2-30.

- Nonionic fatty alcohol alkoxylates like the Neodol surfactants by BASF, the Dobanol surfactants by Shell Corporation or the BioSoft surfactants by Stepan. The alkoxy group being ethoxy, propoxy, butoxy, or mixtures thereof. In addition, these surfactants can be end-capped with methyl groups in order to increase their hydrophobicity.
- Di- and tri-fatty acid chain containing nonionic, anionic and cationic surfactants, and mixtures thereof.
- Fatty acid esters of polyethylene glycol, polypropylene glycol, and polybutylene glycol, or mixtures thereof.
- Polyalphaolefms such as the ExxonMobil PureSym™ PAO line
- Esters such as the ExxonMobil PureSyn™ Esters
- Mineral oil
- Silicone oils such polydimethyl siloxane and polydimethylcyclosiloxane
- Diethyl phthalate
- Di-isodecyl adipate

The level of solvent in the core of the encapsulated fragrance material should be greater than about 30 weight

percent, preferably greater than about 50 weight percent and most preferably greater than about 75 weight percent. In addition to the solvent it is preferred that higher ClogP fragrance materials are employed. It is preferred that greater than about 25 weight percent, preferably greater than 30 and more preferably greater than about 40 weight percent of the fragrance chemicals have ClogP values of greater than about 2.5, preferably greater than about 3 and most preferably greater than about 3.5. Those with skill in the art will appreciate that many formulations can be created employing various solvents and fragrance chemicals. The use of high ClogP fragrance chemicals will require a lower level of hydrophobic solvent than fragrance chemicals with lower ClogP to achieve similar stability. As those with skill in the art will appreciate, in a highly preferred embodiment high ClogP fragrance chemicals and hydrophobic solvents comprise greater than about 80, preferably more than about 90 and most preferably greater than 99 weight percent of the fragrance composition.

It has also been found that the addition of hydrophobic polymers to the core can also improve stability by slowing diffusion of the fragrance from the core. The level of polymer is normally less than 80% of the core by weight, preferably less than 50%, and most preferably less than 20%. The basic requirement for the polymer is that it be miscible or compatible with the other components of the core, namely the fragrance and other solvent. Preferably, the polymer also thickens or gels the core, thus further reducing diffusion. Polymers may be selected from the non-limiting group below:

- Copolymers of ethylene. Copolymers of ethylene and vinyl acetate (Elvax polymers by DOW Corporation). Copolymers of ethylene and vinyl alcohol (EVAL polymers by Kuraray). Ethylene/Acrylic elastomers such as Vamac polymers by Dupont).
- Poly vinyl polymers, such as poly vinyl acetate.
- Alkyl-substituted cellulose, such as ethyl cellulose (Ethocel made by DOW Corporation), hydroxypropyl celluloses (Klucel polymers by Hercules)
- Uncharged polyacrylates. Examples being (i) Amphomer, Demacryl LT and Dermacryl 79, made by National Starch and Chemical Company, (ii) the Amerhold polymers by Amerchol Corporation, and (iii) Acudyne 258 by ISP Corporation.
- Copolymers of acrylic or methacrylic acid and fatty esters of acrylic or methacrylic acid. These are side-chain crystallizing. Typical polymers of this type are those listed in U.S. Patents 4,830,855, 5,665,822, 5,783,302, 6,255,367 and 6,492,462. Examples of such polymers are the Intelimer Polymers, made by Landec Corporation.
- Polypropylene oxide.
- Polybutylene oxide of poly(tetra hydrofuran).
- Polyethylene terephthalate.
- Alkyl esters of poly(methyl vinyl ether) - maleic anhydride copolymers, such as the Gantrez copolymers and Omnirez 2000 by ISP Corporation.
- Carboxylic acid esters of polyamines. Examples of this are ester-terminated polyamide (ETPA) made by Arizona Chemical Company.
- Poly vinyl pyrrolidone (Luviskol series of BASF).
- Block copolymers of ethylene oxide, propylene oxide and/or butylenes oxide. These are known as the Pluronic and Synperonic polymers/dispersants by BASF.
- Another class of polymers include polyethylene oxide-co-propyleneoxide-co-butylene oxide polymers of any ethylene oxide/propylene oxide / butylene oxide ratio with cationic groups resulting in a net theoretical positive charge or equal to zero (amphoteric). The general structure is:

R3-(BuO)z"(PO)y"(EO)x" \ / (EO)x(PO)y(BuO)z-R1
HN-(CH2)y-NH
R4-(BuO)z'''(PO)y'''(EO)x'''/ \(EO)x'(PO)y'(BuO)z'-R2

where R1, 2, 3, 4 is H or any alkyl of fatty alkyl chain group. Examples of such polymers are the commercially known as Tetronics by BASF Corporation.

We have also discovered that when capsules having cores containing a very large proportion of solvents with the appropriate ClogP values and/or with the high ClogP fragrance chemicals described above the encapsulated materials are actually capable of absorbing free fragrance chemicals from product bases.

While it is preferable to have miscible cores within the capsules, the present invention also contemplates the use of emulsion suspension and gel systems that comprise the fragrance or malodour counteracting composition.

Specific examples of malodour counteractant composition components useful in the aminoplast microencapsulates used in the composition and process of our invention are as follows:

**Malodour Counteractant Component Group I:**

1-cyclohexylethan-1-yl butyrate; 1-cyclohexylethan-1-yl acetate; 1-cyclohexylethan-1-ol;
1-(4'-methylethyl)cyclohexylethan-1-yl propionate; and
2'-hydroxy-1'-ethyl(2-phenoxy)acetate, and mixtures thereof,

The above compounds are marketed as VEILEX® by International Flavors & Fragrances Inc., New York, N.Y.

**Malodour Counteractant Component Group II, as disclosed in U.S. Patent 6,379,658:**

β-naphthyl methyl ether; β-naphthyl ketone; benzyl acetone;
mixture of hexahydro-4,7-methanoinden-5-yl propionate and hexahydro-4,7-methanoinden-6-yl propionate; 4-(2,6,6-trimethyl-2-cyclohexen-1-yl)-3-methyl-3-buten-2-one; 3,7-dimethyl-2,6-nonadien-1-nitrile; dodecahydro-3a,6,6,9a-tetramethylnaphtho(2,1-b)furan; ethylene glycol cyclic ester of n-dodecanedioic acid;
1-cyclohexadecen-6-one; 1-cycloheptadecen-10-one; and
corn mint oil.

The beneficial agents of the present invention include such materials as skin moisturizers, skin conditioners, insect repellents, sun tan lotions, ultraviolet light protecting agents, antibacterial agents, hair coloring, hair conditioning, hair cleaning and the like. These agents are well known in the art and are used to those with skill in the art and are used to enhance the appearance, protect and to groom hair and skin. A more complete listing of these materials can be found, for example in the International Cosmetic Ingredient Dictionary and Handbook, Tenth Edition publishes by the Cosmetic, Toiletry and Fragrance Association.

Optionally, in order to provide an increased period of time during which the microencapsulates are retained on the treated human epidermis or on the treated hair follicles, the aminoplast microencapsulates used in the practice of our invention may be coated with a cationic polymer as disclosed in Application for U.S. Letters Patent Serial Number 10/718,240 filed on November 20, 2003 and, in addition, Applications for U.S. Patent 10/268,566 and 10/268,526 filed on October 10, 2002. The rate of use of such cationic polymer coatings on the microencapsulates is from about 1% to about 3000% by weight of the filled microencapsulates; preferably from about 5% to about 1000% by weight of the filled microencapsulates; and most preferably from about 10% to about 500% by weight of the filed microencapsulates.

Examples of such cationic polymers used as coatings are cationically modified starch and cationically modified guar, polymers comprising poly diallyl dimethyl ammonium halides (PolyDADMAC), and copolymers of DADMAC with vinyl pyrrolidone, acrylamides, imidazoles, imidazolinium halides, and the like. For instance, Polyquaternium-6, 7, 22 and 39, all available from Ondeo Nalco.

The preferred cationic starch has a molecular weight of from about 100,000 to about 500,000,000, preferably from about 200,000 to about 10,000,000 and most preferably from about 250,000 to about 5,000,000. The preferred cationic starch products are HI-CAT CWS42 and HI-CAT 02 and are commercially available from ROQUETTE AMERICA, Inc.

The preferred cationic guar has a molecular weight of from about 50,000 to about 5,000,000. The preferred cationic guar products are Jaguar C-162 and Jaguar C-17 and are commercially available from Rhodia Inc.

Additional examples of cationic polymers useful for coating the encapsulated solvent/fragrance and solvent/malodour counteractant compositions of our invention are the water-soluble cationic amino resins, cationic urea resins, specifically, urea-formaldehyde prepolymers subjected to polycondensation with a cationic modifier such as diethylenetriamine, tetraethylene pentamine, guanidine, guanyl urea and oxazolidine as disclosed in published U.S. Patent Application US 2001/008874 A1 published on July 19, 2001, for example U-RAMIN P-1500, Mitsui Kagaku K.K., Tokyo Japan a urea-formaldehyde pre-polymer modified with diethylene triamine.

Once the fragrance material and malodour counteractant is encapsulated a cationically charged water-soluble polymer is applied to the fragrance encapsulated polymer. This water-soluble polymer can also be an amphoteric polymer with a ratio of cationic and anionic functionalities resulting in a net total charge of zero and positive, i.e., cationic. Those skilled in the art would appreciate that the charge of these polymers can be adjusted by changing the pH, depending on the product in which this technology is to be used. Any suitable method for coating the cationically

charged materials onto the encapsulated fragrance materials can be used. The nature of suitable cationically charged polymers for assisted capsule delivery to interfaces depends on the compatibility with the capsule wall chemistry since there has to be some association to the capsule wall. This association can be through physical interactions, such as hydrogen bonding, ionic interactions, hydrophobic interactions, electron transfer interactions or, alternatively, the polymer coating could be chemically (covalently) grafted to the capsule or particle surface. Chemical modification of the capsule or particle surface is another way to optimize anchoring of the polymer coating to capsule or particle surface. Furthermore, the capsule and the polymer need to want to go to the desired interface and, therefore, need to be compatible with the chemistry (polarity, for instance) of that interface. Therefore, depending on which capsule chemistry and interface (e.g., cotton, polyester, hair, skin, wool) is used the cationic polymer can be selected from one or more polymers with an overall zero (amphoteric: mixture of cationic and anionic functional groups) or net positive charge, based on the following polymer backbones: polysaccharides, polypeptides, polycarbonates, polyesters, polyolefinic (vinyl, acrylic, acrylamide, poly diene), polyester, polyether, polyurethane, polyoxazoline, polyamine, silicone, polyphosphazine, olyaromatic, poly heterocyclic, or polyionene, with molecular weight (MW) ranging from about 1,000 to about 1000,000,000, preferably from about 5,000 to about 10,000,000. As used herein molecular weight is provided as weight average molecular weight. Optionally, these cationic polymers can be used in combination with nonionic and anionic polymers and surfactants, possibly through coacervate formation.

A more detailed list of cationic polymers that can be used to coat the encapsulated fragrance and malodour counteractant composition is provided below:

Polysaccharides include but are not limited to guar, alginates, starch, xanthan, chitosan, cellulose, dextrans, arabic gum, carrageenan, hyaluronates. These polysaccharides can be employed with:

(a) cationic modification and alkoxy-cationic modifications, such as cationic hydroxyethyl, cationic hydroxy propyl. For example, cationic reagents of choice are 3-chloro-2-hydroxypropyl trimethylammonium chloride or its epoxy version. Another example is graft-copolymers of polyDADMAC on cellulose like in Celquat L-200 (Polyquaternium-4), Polyquatemium-10 and Polyquaternium-24, commercially available from National Starch, Bridgewater, N.J.;
(b) aldehyde, carboxyl, succinate, acetate, alkyl, amide, sulfonate, ethoxy, propoxy, butoxy, and combinations of these functionalities. Any combination of Amylose and Amylopectin and overall molecular weight of the polysaccharide; and
(c) any hydrophobic modification (compared to the polarity of the polysaccharide backbone).

The above modifications described in (a), (b) and (c) can be in any ratio and the degree of functionalization up to complete substitution of all functionalizable groups, and as long as the theoretical net charge of the polymer is zero (mixture of cationic and anionic functional groups) or preferably positive. Furthermore, up to 5 different types of functional groups may be attached to the polysaccharides. Also, polymer graft chains may be differently modified than the backbone. The counterions can be any halide ion or organic counter ion. See, for example, U.S. Patent Nos. 6,297,203 and 6,200,554.

Another source of cationic polymers contain protonatable amine groups so that the overall net charge is zero (amphoteric: mixture of cationic and anionic functional groups) or positive. The pH during use will determine the overall net charge of the polymer. Examples are silk protein, zein, gelatin, keratin, collagen and any polypeptide, such as polylysine.

Further cationic polymers include poly vinyl polymers, with up to 5 different types of monomers, having the monomer generic formula -C(R2)(R1)-CR2R3-. Any co-monomer from the types listed in this specification may also be used. The overall polymer will have a net theoretical positive charge or equal to zero (mixture of cationic and anionic functional groups). Where R1 is any alkanes from C1-C25 or H; the number of double bonds ranges from 0-5. Furthermore, R1 can be an alkoxylated fatty alcohol with any alkoxy carbon-length, number of alkoxy groups and C1-C25 alkyl chain length. R1 can also be a liquid crystalline moiety that can render the polymer thermotropic liquid crystalline properties, or the alkanes selected can result in side-chain melting. In the above formula R2 is H or CH3; and R3 is -Cl, -NH2 (i.e., poly vinyl amine or its copolymers with N-vinyl formamide. These are sold under the name Lupamin 9095 by BASF Corporation), -NHR1, -NR1R2, -NR1R2 R6 (where R6 = R1, R2, or -CH2-COOH or its salt), -NH-C(O)-H, -C(O)-NH2 (amide), -C(O)-N(R2)(R2')(R2"), -OH, styrene sulfonate, pyridine, pyridine-N-oxide, quaternized pyridine, imidazolinium halide, imidazolium halide, imidazol, piperidine, pyrrolidone, alkyl-substituted pyrrolidone, caprolactam or pyridine, phenyl-R4 or naphthalene-R5 where R4 and R5 are R1, R2, R3, sulfonic acid or its alkali salt -COOH, - COO- alkali salt, ethoxy sulphate or any other organic counter ion. Any mixture or these R3 groups may be used. Further suitable cationic polymers containing hydroxy alkyl vinyl amine units, as disclosed in U.S. Patent No 6,057,404.

Another class of materials are polyacrylates, with up to 5 different types of monomers, having the monomer generic formula: -CH(R1)-C(R2)(CO-R3-R4)-. Any co-monomer from the types listed in this specification may also be

used. The overall polymer will have a net theoretical positive charge or equal to zero (mixture of cationic and anionic functional groups). In the above formula R1 is any alkane from C1-C25 or H with number of double bonds from 0-5,aromatic moieties, polysiloxane, or mixtures thereof. Furthermore, R1 can be an alkoxylated fatty alcohol with any alkoxy carbon-length, number of alkoxy groups and C1-C25 alkyl chain length. R1 can also be a liquid crystalline moiety that can render the polymer thermotropic liquid crystalline properties, or the alkanes selected can result in side-chain melting. R2 is H or CH3; R3 is alkyl alcohol C1-25 or an alkylene oxide with any number of double bonds, or R3 may be absent such that the C=O bond is (via the C-atom) directly connected to R4. R4 can be: -NH2, NHR1, - NR1R2, -NR1R2 R6 (where R6 = R1, R2, or -CH2-COOH or its salt), -NH-C(O)-, sulfo betaine, betaine, polyethylene oxide, poly(ethyleneoxide/propylene oxide/butylene oxide) grafts with any end group, H, OH, styrene sulfonate, pyridine, quaternized pyridine, alkyl-substituted pyrrolidone or pyridine, pyridine-N-oxide, imidazolinium halide, imidazolium halide, imidazol, piperidine, - OR1, -OH, -COOH alkali salt, sulfonate, ethoxy sulphate, pyrrolidone, caprolactam, phenyl-R4 or naphthalene-R5 where R4 and R5 are R1, R2, R3, sulfonic acid or its alkali salt or organic counter ion. Any mixture or these R3 groups may be used. Also, glyoxylated cationic polyacrylamides can be used. Typical polymers of choice are those containing the cationic monomer dimethylaminoethyl methacrylate (DMAEMA) or methacrylamidopropyl trimethyl ammonium chloride (MAPTAC). DMAEMA can be found in Gafquat and Gaffix VC-713 polymers from ISP. MAPTAC can be found in BASF's Luviquat PQ11 PN and ISP's Gafquat

HS 100.

Another group of polymers that can be used are those that contain cationic groups in the main chain or backbone. Included in this group are:

(1) polyalkylene imines such as polyethylene imine, commercially available as Lupasol from BASF. Any molecular weight and any degree of crosslinking of this polymer can be used in the present invention;
(2) ionenes having the general formula set forth as -[ N(+)R1R2-A1-N(R5)-X-N(R6)-A2-N(+)R3R4-A3 ]n- 2Z-, as disclosed in U.S. Patent Nos. 4,395,541 and U.S. 4,597,962;
(3) adipic acid/dimethyl amino hydroxypropyl diethylene triamine copolymers, such as Cartaretin F-4 and F-23, commercially available from Sandoz;
(4) polymers of the general formula-[N(CH3)2-(CH2)x-NH-(CO)-NH-(CH2)y- N(CH3)2)-(CH2)z-O-(CH2)p]n-, with x, y, z, p=1-12, and n according to the molecular weight requirements. Examples are Polyquaternium 2 (Mirapol A-15), Polyquaternium-17 (Mirapol AD-1), and Polyquaternium-18 (Mirapol AZ-1).

Other polymers include cationic polysiloxanes and cationic polysiloxanes with carbon-based grafts with a net theoretical positive charge or equal to zero (mixture of cationic and anionic functional groups). This includes cationic end-group functionalized silicones (i.e,. Polyquaternium-80). Silicones with general structure: -[-Si(R1)(R2)-O-]x-[Si (R3)(R2)-O-]y-where R1 is any alkane from C1-C25 or H with number of double bonds from 0-5,aromatic moieties, polysiloxane grafts, or mixtures thereof. R1 can also be a liquid crystalline moiety that can render the polymer thermotropic liquid crystalline properties, or the alkanes selected can result in side-chain melting. R2 can be H or CH3 and R3 can be -R1-R4, where R4 can be -NH2, -NHR1, -NR1R2, -NR1R2R6 (where R6 = R1, R2, or -CH2-COOH or its salt), -NH-C(O)-, - COOH, -COO- alkali salt, any C1-25 alcohol, -C(O)-NH2 (amide), -C(O)-N(R2)(R2')(R2''), sulfo betaine, betaine, polyethylene oxide, poly(ethyleneoxide/propylene oxide/butylene oxide) grafts with any end group, H, -OH, styrene sulfonate, pyridine, quaternized pyridine, alkyl-substituted pyrrolidone or pyridine, pyridine-N-oxide, imidazolinium halide, imidazolium halide, imidazol, piperidine, pyrrolidone, caprolactam, -COOH, -COO- alkali salt, sulfonate, ethoxy sulphate phenyl-R5 or naphthalene-R6 where R5 and R6 are R1, R2, R3, sulfonic acid or its alkali salt or organic counter ion. R3 can also be -(CH2)x-O-CH2-CH(OH)-CH2-N(CH3)2-CH2-COOH and its salts. Any mixture of these R3 groups can be selected. X and y can be varied as long as the theoretical net charge of the polymer is zero (amphoteric) or positive. In addition, polysiloxanes containing up to 5 different types of monomeric units may be used. Examples of suitable polysiloxanes are found in U.S. Patent Nos. 4,395,541 4,597,962 and 6,200,554. Another group of polymers that can be used to improve capsule/particle deposition are phospholipids that are modified with cationic polysiloxanes. Examples of these polymers are found in U.S. Patent No. 5,849,313, WO Patent Application 9518096A1 and European Patent EP0737183B1.

Furthermore, copolymers of silicones and polysaccharides and proteins can be used (Crodasone Series).

Another class of polymers include polyethylene oxide-co-propyleneoxide-co-butylene oxide polymers of any ethylene oxide/propylene oxide / butylene oxide ratio with cationic groups resulting in a net theoretical positive charge or equal to zero (amphoteric). The general structure is:

R3-(BuO)z"(PO)y"(EO)x" \ / (EO)x(PO)y(BuO)z-R1
(R5)N-(CH2)y-N(R6)
R4-(BuO)z'''(PO)y'''(EO)x'''/ \ (EO)x'(PO)y'(BuO)z'-R2

where R1,2,3,4 is NH2, -N(R)3- X+, R with R being H or any alkyl group. R5,6 is -CH3 or H. Counter ions can be any halide ion or organic counter ion. X, Y, may be any integer, any distribution with an average and a standard deviation and all 12 can be different. Examples of such polymers are the commercially available TETRONIC brand polymers.

Suitable polyheterocyclic (the different molecules appearing in the backbone) polymers include the piperazine-alkylene main chain copolymers disclosed in Ind. Eng. Chem. Fundam., (1986), 25, pp.120-125, by Isamu Kashiki and Akira Suzuki.

Also suitable for use in the present invention are copolymers containing monomers with cationic charge in the primary polymer chain. Up to 5 different types of monomers may be used. Any co-monomer from the types listed in this specification may also be used. Examples of such polymers are poly diallyl dimethyl ammonium halides (PolyDADMAC) copolymers of DADMAC with vinyl pyrrolidone, acrylamides, imidazoles, imidazolinium halides, etc. These polymers are disclosed in Henkel EP0327927A2 and PCT Patent Application 01/62376A1. Also suitable are Polyquaternium-6 (Merquat 100), Polyquaternium-7 (Merquats S, 550, and 2200), Polyquaternium-22 (Merquats 280 and 295) and Polyquaternium-39 (Merquat Plus 3330), available from Ondeo Nalco.

The preferred cationic starch has a molecular weight of from about 100,000 to about 500,000,000, preferably from about 200,000 to about 10,000,000 and most preferably from about 250,000 to about 5,000,000. The preferred cationic starch products are HI-CAT CWS42 and HI-CAT 02 and are commercially available from ROQUETTE AMERICA, Inc.

The preferred cationic guar has a molecular weight of from about 50,000 to about 5,000,000. The preferred cationic guar products are Jaguar C-162 and Jaguar C-17 and are commercially available from Rhodia Inc.

## The Base

The base in which the aminoplast microencapsulates of solvent/fragrance compositions and/or solvent/malodour counteractant compositions used in the practice of our invention are suspended is a solution, a powder such as talc; a gel base or an emulsion base. The concentration of fragrance composition components and/or malodour counteractant composition components in the base is in the range of from about 0.5% to about 50% by weight of the human epidermal treatment and/or hair treatment composition of our invention. The preferable concentration range when using an emulsion base or gel is from about 0.5% to about 10 %.

In the case where the human epidermal skin and/or hair treatment base is an emulsion base, the pH of the emulsion base is in the range of from about 4.5 to about 9, and the preferred constituents of the emulsion base are water, at least one suspending agent, at least one pH buffering agent, at least one emulsifier, at least one emulsion stabilizer and at least one moisturizer. Preferred emulsifiers are (i) the polyethylene glycol ester of stearic acid,

PEG(100)monostearate, $CH_3(CH_2)_{16}C{=}O$
|
$(OCH_2CH_2)_nOH$

wherein the average value of n is 100, marketed under the name: MYRJ 68, trademark of ICI Americas of Wilmington, Delaware 19803-8340, U.S.A;(ii) triethanolamine-mono stearate; and (iii)polyethylene glycol(20)sorbitan monostearate marketed under the name, TWEEN 60, trademark of ICI Americas Inc., Bridgewater, New Jersey. A preferred pH buffering agent is triethanolamine. Preferred suspending agents are those set forth in U.S. Patent 4,428,869 and include xanthan gum, guar gum, attapulgite clay, hydroxypropyl cellulose having a molecular weight of from about 50,000 to about 800,000, colloidal silica, and ethyl cellulose having a particle size of from about 0.004 to about 0.130 microns, a surface area of from about 100 to about 500 $m^2$ per gram and a density of from about 1.0 to about 4.0 pounds per cubic foot. Preferred moisturizers are (i) glycerine, (ii) lanolin alcohol-mineral oil compositions, (iii) propylene glycol and (iv) *Butyrospermum parkii* ("Shea Butter"). A preferred emulsion stabilizer is the polymer of acrylic acid crosslinked with an allyl ether of sucrose, Carbopol 934" marketed as ACRITAMER 934, R.I.T.A. Corporation, Woodstock, Illinois. Additional optional ingredients for the emulsion base are solvents, such as cyclomethicone including SILICONE 344 by Dow-Corning, ultra-violet radiation absorbers, e.g., octyl methoxy cinnamate, viscosity control agents, e.g.,

cetyl alcohol and preservatives, e.g., propyl paraben (n-propyl-p-hydroxybenzoate).

In the case where the human epidermal skin and/or hair treatment base is a gel base, the pH of the gel base is in the range of from about 4.5 to about 9.0 and the preferred constituents of the gel base are water, at least one gelling agent, at least one suspending agent, at least one pH buffering agent and at least one moisturizer. A preferred gelling agent is the polymer of acrylic acid cross-linked with an allyl ether of sucrose , Carbopol 934. Preferred suspending agents are those set forth in U.S. Patent 4,428,869 and include xanthan gum, guar gum, attapulgite clay, hydroxypropyl cellulose having a molecular weight of from about 50,000 to about 800,000, colloidal silica, and ethyl cellulose having a particle size of from about 0.004 to about 0.130 microns, a surface area of from about 100 to about 500 $m^2$ per gram and a density of from about 1.0 to about 4.0 pounds per cubic foot. Preferred moisturizers are (i) glycerine, (ii) lanolin alcohol-mineral oil compositions, (iii) propylene glycol and (iv) *Butyrospermum parkii* ("Shea Butter"). A preferred pH buffering agent is triethanolamine. Additional optional ingredients for the gel base are preservatives e.g., propyl paraben (n-propyl-p-hydroxybenzoate) and 'skin-feel' agents or 'slip' agents, for example, a 3% aqueous solution of polyethylene glycol-14,000 having the formula: $H(OCH_2CH_2)_nOH$ where n = 14,000, marketed as POLYOX WSR-N-3000, Union Carbide Chemicals & Plastics Technology Corporation, Danbury, Connecticut.

The human epidermal and/or fair treatment composition of our invention thus formed is then used in the process of our invention which comprises the step of supplying to a given region of the human epidermis and/or a group of human hair follicles a human epidermis-treating quantity and/or a human hair-treating quantity of the treatment composition over a human epidermis treatment and/or human hair treatment period of time, preferably over a 10 second to 30 second period of time. The application is carried out using any applicator known to those skilled in the art, for example, the dispenser described in U.S. Patent 4,708,267.

The following Table II sets forth skin or hair treatment processes and compositions disclosed in U.S. Patents and published U.S. Patent Applications where the human epidermal and/or hair treatment compositions of our invention may be used in conjunction therewith:

TABLE II

| U.S. Patent Number or Published Patent Application Number | Nature of Use and/or Title |
|---|---|
| U.S. Patent 5,460,805 | Body powder comprising colorant |
| U.S. Patent 6,348,218 | Self dosing skin preparation |
| U.S. Patent 6,479,042 | Hair setting composition |
| U.S. Patent 6,491,933 | Skin and hair cleansing composition |
| U.S. Patent 6,479,059 | Scalp treatment composition |
| U.S. Patent 6,491,902 | Hair treatment composition |
| U.S. Patent 6,497,860 | Skin lightening compositions |
| U.S. Patent 6,479,043 | Depilatory composition |
| U.S. Patent 6,592,857 | Cosmetic cream including tertiary amide-terminated polyamide |
| U.S. Patent 6,485,713 | Sunscreen compositions |
| U.S. Patent 6,531,160 | Day cream composition |
| U.S. Patent 6,649,154 | Hairdressing cosmetic preparation |
| U.S. Patent 6,649,173 | Topical composition comprising fluorinated silicone compound |
| U.S. Patent 6,649,175 | Skin barrier composition |
| U.S. Patent 6,649,178 | Cosmetic composition for stressed skin |
| U.S. Patent 6,649,578 | Process for cleaning and conditioning hair and skin |
| U.S. Patent 6,649,579 | Soy based hand cleaner |
| Published U.S. Patent Application US 2003/0180335 A1 published 9/25/03 | Skin treatment composition |

Optionally, the human epidermal and/or hair treatment composition of our invention may contain non-confined fragrance. The advantage of use of the non-confined fragrance is that immediately subsequent to application of the treatment composition to a region of the human epidermis or to a group of hair follicles in accordance with the process of our invention, an initial 'burst' of fragrance occurs. A secondary burst of fragrance and/or malodour counteractant occurs at the time of rubbing the human epidermis or at the time of hair brushing, as the case may be, in the treatment region where the microcapsules are left on or remain after treatment. Controlled release of fragrance composition and/or the malodour counteractant composition occurs over a relatively long period of time in the treatment region where the microcapsules remain. The C $log_{10}P$ range of each of the non-confined fragrance components is in the range of from about 1 to about 8 thus enabling a greater range of fragrance component types in the non-confined fragrance as opposed to the components of the confined fragrance.

Specific examples of non-confined fragrance components, their molecular weights and their C $log_{10}P$'s are set forth in the following Table III:

TABLE III

| **Fragance Component** | **$Clog_{10}P$ value** | **Molecular Weight** |
|---|---|---|
| benzaldehyde | 1.480 | 106.12 |
| benzyl acetate | 1.960 | 150.17 |
| laevo-carvone | 2.083 | 150.22 |
| geraniol | 2.649 | 154.26 |
| cis-jasmone | 2.712 | 164.25 |
| β-phenylethyl alcohol | 1.183 | 122.17 |
| α-terpineol | 2.569 | 154.25 |
| δ-nonalactone | 2.760 | 156.23 |
| 1-phenyl hexanol-5 | 3.299 | 178.28 |
| dihydromyrcenol | 3.03 | 156.27 |
| δ-undecalactone | 3.830 | 184.28 |
| amyl cinnamate | 3.771 | 218.30 |
| benzophenone | 3.120 | 182.22 |
| nerol | 2.649 | 154.25 |
| 2-methoxynaphthalene | 3.235 | 158.20 |
| ethyl undecylenate | 4.888 | 212.34 |
| geranyl anthranilate | 4.216 | 273.38 |
| α-irone | 3.820 | 206.33 |
| α-santalol | 3.800 | 220.36 |
| iso-eugenol | 2.547 | 164.21 |
| amyl salicylate | 4.601 | 208.26 |
| benzyl salicylate | 4.383 | 228.25 |
| β-caryophyllene | 6.333 | 204.36 |
| cedrol | 4.530 | 222.37 |
| cedryl acetate | 5.436 | 264.41 |
| cedryl formate | 5.070 | 238.37 |
| cyclohexyl salicylate | 5.265 | 220.29 |
| γ-dodecalactone | 4.359 | 198.31 |
| ethyl undecylenate | 4.888 | 212.34 |

TABLE III (continued)

| Fragance Component | $Clog_{10}P$ value | Molecular Weight |
|---|---|---|
| geranyl anthranilate | 4.216 | 273.38 |
| β-phenylethyl benzoate | 4.058 | 226.38 |
| β-phenylethyl phenyl acetate | 3.767 | 240.31 |
| 5-acetyl-1,1,2,3,3,6-hexamethyl indane | 5.977 | 258.41 |
| cyclopentadecanolide | 6.246 | 240.39 |
| d-limonene | 4.232 | 136.24 |
| cis-p-t-butylcyclohexyl acetate | 4.019 | 198.31 |
| amyl cinnamic aldehyde | 4.324 | 202.30 |

## BRIEF DESCRIPTION OF THE DRAWINGS

Figure 1 is a perspective view, on a greatly enlarged scale of a fragrance and/or malodour counteractant-containing microcapsule useful in the practice of our invention.

Figure 2 is a cross-sectional view of a first embodiment of the microcapsule of Figure 1.

Figure 3 is a cross-sectional view of a second embodiment of the microcapsule of Figure 1 being coated with a cationic polymer coating.

Figure 4a is a cross-sectional view of a third embodiment of the microcapsule of Figure 1, showing in schematic form, a first mechanism for formation of the microcapsule wall.

Figure 4b is a cross-sectional view of a third embodiment of the microcapsule of Figure 1, showing in schematic form, a second mechanism for formation of the microcapsule wall.

Figure 5 is a graph showing measured release % at a 1 week period vs. measured $log_{10}P$ for fragrance components contained in the microcapsules used in the practice of our invention, where P is the n-octanol/water partition coefficient for each of the fragrance components.

Figure 6 is a schematic block-flow diagram showing, in schematic form, a process of our invention including fragrance composition and/or malodour counteractant composition microencapsulation, suspension of the resulting microcapsules in a gel or emulsion base, and packaging of the resulting product.

Figure 7 is a set of bar graphs of perceived intensity (on a scale of 0-20 as measured on the "Y" axis) vs. time (in hours, as measured on the "X" axis) for a microencapsulated fragrance as used in the practice of our invention and for a non-confined fragrance, deposited on the human epidermis, in the absence of rubbing.

Figure 8 is a set of bar graphs of perceived intensity (on a scale of 0-20 as measured on the "Y" axis) vs. time (in hours, as measured on the "X" axis) for a microencapsulated fragrance as used in the practice of our invention and for a non-confined fragrance, deposited on the human epidermis, with rubbing taking place at the 8 and at the 12 hour time period.

Figure 9 is a set of graphs summarizing the data of each of Figure 7 and of Figure 8 with perceived intensity (on a scale of 0-20 as measured on the "Y" axis) vs. time (in hours, as measured on the "X" axis). In each case, the mathematical model is in accordance with the algorithm:

$$Y = A \text{ Log }_e X + B$$

wherein Y is a scaled measure of fragrance intensity on a scale of 0 - 20 and X is time as measured in hours subsequent to the application of the human epidermal treatment composition; and wherein A is in the range of from -3.0 to -8.0 and B is in the range of from 20 to 26. For the case where the fragrance is microencapsulated, A is in the range of from -3.0 to - 8.0 and B is in the range of from 22 to 26.

## DETAILED DESCRIPTION OF THE DRAWINGS

Each of the microcapsules of Figures 1, 2, 3, 4a and 4b is designated in its entirety by the reference numeral 4. Microcapsule 4 comprises an aminoplast shell 6 having a fill 8 therewith which fill comprises a fragrance composition and/or a malodour counteractant composition in admixture with a solvent as described supra and exemplified infra. Figure 3 shows the microcapsule coated with a cationic polymer, with the cationic polymer coating indicated by reference numeral 9. As indicated supra, such cationic polymers are disclosed in Applications for U.S. Patent 10/268,566 and 10/268,526 filed on October 10, 2002. Figures 4a and 4b indicate, schematically, the formation of the polymeric aminoplast shell by means of interfacial polymerization reaction of a melamine-formaldehyde precondensate, e.g., URAC 180 (shown as "X") with an acrylic acid polymer or copolymer (shown as "Y") thereby forming the cross-linked acrylic acid polymer or copolymer ("-(-X-Y-)$_N$-"). In Figure 4a, the melamine formaldehyde precondensate is initially contained in the solvent-fragrance and/or malodour counteractant phase, 4 and the acrylic acid polymer or co-polymer is dispersed in an aqueous phase. Reaction takes place at location 6 where the cross-linked acrylic acid polymer or copolymer (the 'aminoplast resin' ) is formed. Curing at higher temperatures, e.g., 50 - 85°C then takes place at location 6. In Figure 4b each of the melamine-formaldehyde precondensate and the acrylic acid polymer or co-polymer is dispersed in the aqueous phase and reacted under acidic conditions. The resulting un-cured cross-linked acrylic acid polymer or co-polymer coacervates about the solvent-fragrance and/or malodour counteractant phase droplets at location 6. Curing at higher temperatures, e.g., 50 - 85°C then takes place at location 6.

Referring to Figure 5, the measured fragrance release % after storage of fragrance component-containing aminoplast microcapsules (having shell walls composed of an acrylamide-acrylic acid co-polymer cross-linked with a melamine-formaldehyde pre-condensate) at 25°C for a period of 1 week is measured along the "Y" axis indicated by reference numeral 50, with the measured $\log_{10}P$ plotted on the "X" axis, indicated by reference numeral 51. A linear regression function, **Y = -0.225X + 1.949** for data points 52a is indicated by reference numeral 52.

Referring to Figure 6, fragrance component and/or malodour counteractant component formulation in storage tank 61 is passed through line 63 past control valve 64 into mixing vessel 67 equipped with agitator means 68, whereat is admixed with solvent, e.g., NEOBEE-M5 stored in tank 62 from which it is passed through line 65 past control valve 66 into mixing vessel 67 where an oil-phase, monophasic solvent/fragrance formulation and/or solvent/malodour counteractant formulation is prepared. Similtaeneously, (i) substituted or un-substituted acrylic acid polymer or co-polymer, e.g., an acrylic acid-acrylamide co-polymer, stored in tank 84 is passed through line 85 past control valve 86 into reactor 87 equipped with agitator means 88 and heating supply 89; and (ii) urea or melamine in storage vessel 69 is passed through line 71 past control valve 74 to reactor 75 where it is reacted with formaldehyde or formalin stored in tank 70 and passed through line 72 past control valve 73 into reactor 75, in order to form a urea-formaldehyde pre-condensate or melamine-formaldehyde pre-condensate which, in turn, is passed through line 79 past control valve 82 into reactor 87 (optionally, the pre-condensate may be partially etherified with a $C_1$-$C_6$ alkanol, in a reactor, not shown, located between valve 82 and reactor 87) together with water, originally stored in vessel 76, which is passed through line 80 past control valve 81, and aqueous acid pH adjustment composition which is originally stored in vessel 77, which is passed through line 78 past control valve 83. An uncured substituted or un-substituted acrylic acid polymer or co-polymer, cross-linked with the urea-formaldehyde pre-condensate or melamine-formaldehyde pre-condensate is formed in reactor 87. A mixture of water and the resulting uncured substituted or un-substituted acrylic acid polymer or co-polymer, cross-linked with the urea-formaldehyde pre-condensate or melamine-formaldehyde pre-condensate is passed through line 92 past control valve 93 into homogenzer 94 where it is vigorously admixed with the solvent/fragrance formulation and/or solvent/malodour counteractant formulation which is passed from vessel 67 through line 90 past control valve 91. The homogenizer is preferably of a type illustrated in Figures 11-A and 11-B of U.S. Patent 6,042,792 and described therein. During the homogenization unit process, the un-cured substituted or un-substituted acrylic acid polymer or co-polymer, cross-linked with the urea-formaldehyde pre-condensate or melamine-formaldehyde pre-condensate (which is originally dispersed in the aqueous phase) is coacervated about each of the monophasic oil-phase droplets of the solvent/fragrance formulation and/or solvent/malodour counteractant formulation thereby forming filled microcapsules having uncured microcapsule shell walls, as shown in Figure 4b, described supra. The resulting uncured filled micropsules in an aqueous slurry are passed from homogenizer 94 through line 102 past control valve 103 into curing vessel 104 which is equipped with heating means 110, and whereat the uncured filled microcapsules are cured at 50 - 85°C. Optionally, subsequent to the curing unit process, cationic coating polymer (e.g., U-RAMIN P-1500) , stored in vessel 105 is passed through line 106 past control valve 107 into curing vessel 104 where the cationic polymer is coated onto the outer surface of each filled cured microcapsule. The cured, filled, optionally-coated

microcapsule slurry is then passed through line 111 into tank 108 equipped with agitator means 109 where it is mixed with the gel base or emulsion base stored in tank 100 and passed through line 101 into tank 108, simultaneously with suspension agent which either may be stored at location 98 and conveyed into tank 108 through line 99 and/or combined with the gel base or emulsion base in tank 100 by means of passing the suspension agent through line 118 past control valve 119 into tank 100. Optionally, non-confined fragrance, stored in tank 95 is (i) passed through line 96 past control valve 97 into tank 108 and/or (ii) passed through line 116 past control valve 117 into tank 100 whereat it is admixed with the gel base or emulsion base stored therein. The resulting slurry, stored in tank 108 is conveyed via conveyance 112 to packaging location 113 whereat it is packaged and forwarded via marketing channels 115 for marketing and use epidermal and/or hair application utilities, 114.

In Figure 7, showing the results of Example III, the set of bar graphs of perceived intensity (on a scale of 0-20 as measured on the "Y" axis indicated by reference numeral 120) vs. time (in hours, as measured on the "X" axis indicated by reference numeral 121) is for a microencapsulated fragrance (the fragrance of Example A microencapsulated in microcapsules, each of which has a wall composed of an acrylic acid-acrylamide co-polymer cross-linked with a melamine-formaldehyde pre-condensate as described in Example I, infra, with each of the microcapsules suspended in the gel base of Example II, infra) used in the practice of our invention (shown by bar graphs 122b, 123b and 124b where the standard deviation for the perceived intensity values are, respectively, 122d, 123d and 124d) and for a non-confined fragrance (the fragrance of Example A), (shown by bar graphs 122a, 123a and 124a where the standard deviation for each of the perceived intensity values are, respectively, 122c, 123c and 124c), deposited on the human epidermis, in the absence of rubbing.

In Figure 8 showing the results of Example IV, the set of bar graphs of perceived intensity (on a scale of 0-20 as measured on the "Y" axis indicated by reference numeral 120) vs. time (in hours, as measured on the "X" axis indicated by reference numeral 121) is for a microencapsulated fragrance (the fragrance of Example A microencapsulated in microcapsules, each of which has a wall composed of an acrylic acid-acrylamide co-polymer cross-linked with a melamine-formaldehyde pre-condensate as described in Example I, infra, with each of the microcapsules suspended in the gel base of Example II, infra) used in the practice of our invention (shown by bar graphs 125b, 126b and 127b where the standard deviation for the perceived intensity values are, respectively, 125d, 126d and 127d) and for a non-confined fragrance (the fragrance of Example A), (shown by bar graphs 125a, 126a and 127a where the standard deviation for each of the perceived intensity values are, respectively, 125c, 126c and 127c), deposited on the human epidermis with rubbing taking place at the 8 and at the 12 hour time period.

In Figure 9, the set of graphs summarizing the data of each of Figure 7 and of Figure 8 with perceived intensity (on a scale of 0-20 as measured on the "Y" axis indicated by reference numeral 202) vs. time (in hours, as measured on the "X" axis indicated by reference numeral 201) is indicated by reference numerals 203 and 204 for the bar graphs of Figure 7 and by reference numerals 205 and 206 for the bar graphs of Figure 8.

For the case of the bar graphs for the non-confined fragrance of Figure 7, indicated by reference numerals 122a, 123a and 124a, for the graph indicated by reference numeral 203, the mathematical model is in accordance with the algorithm, $\mathbf{Y = -5.8 \, Log_e \, X + 20.9}$.

For the case of the bar graphs for the microencapsulated fragrance of Figure 7, indicated by reference numerals 122b, 123b and 124b, for the graph indicated by reference numeral 204, the mathematical model is in accordance with the algorithm, $\mathbf{Y = -7.23 \, Log_e \, X + 25.9}$.

For the case of the bar graphs for the non-confined fragrance of Figure 8, indicated by reference numerals 125a, 126a and 127a, for the graph indicated by reference numeral 205, the mathematical model is in accordance with the algorithm, $\mathbf{Y = -5.53 \, Log_e \, X + 20.62}$.

For the case of the bar graphs for the microencapsulated fragrance of Figure 8, indicated by reference numerals 125b, 126b and 127b, for the graph indicated by reference numeral 206, the mathematical model is in accordance with the algorithm, $\mathbf{Y = -3.82 \, Log_e \, X + 22.5}$.

In summary for each case, the mathematical model is in accordance with the algorithm:

$$Y = A \, Log_e X + B$$

wherein Y is a scaled measure of fragrance intensity on a scale of 0 - 20 and X is time as measured in hours subsequent to the application of the human epidermal treatment composition; and wherein A is in the range of from -3.0 to -8.0 and B is in the range of from 20 to 26.

For the case where the fragrance is microencapsulated, A is in the range of from -3.0 to -8.0 and B is in the range of from 22 to 26.

The following examples are not meant to define or otherwise limit the scope of the invention. Rather the scope of the invention is to be ascertained according to the claims that follow the examples. Unless noted to the contrary, all percentages are given on a weight percent on a dry basis.

**EXAMPLE A**

The following fragrance composition was prepared:

| Fragrance Component | C $\log_{10}P$ value | Molecular Weight | Parts by Weight |
|---|---|---|---|
| ethyl undecylenate | 4.888 | 212.34 | 3.0 |
| geranyl anthranilate | 4.216 | 273.38 | 7.5 |
| α-irone | 3.820 | 206.33 | 6.3 |
| phenyl ethyl benzoate | 4.058 | 226.28 | 3.2 |
| d-limonene | 4.232 | 136.24 | 3.2 |
| cis-p-t-butylcyclohexyl acetate | 4.019 | 198.31 | 5.8 |
| amyl cinnamic aldehyde | 4.324 | 202.30 | 7.3 |
| hexyl cinnamic aldehyde | 5.473 | 216.33 | 12.6 |
| hexyl salicylate | 5.260 | 222.29 | 12.6 |

**EXAMPLE B**

The following fragrance composition was prepared:

| Fragrance Component | C $\log_{10}P$ value | Molecular Weight | Parts by Weight |
|---|---|---|---|
| β-phenyl ethanol | 1.183 | 122.17 | 13.0 |
| benzyl acetate | 1.960 | 150.17 | 17.5 |
| α-irone | 3.820 | 206.33 | 6.3 |
| phenyl ethyl benzoate | 4.058 | 226.28 | 3.2 |
| d-limonene | 4.232 | 136.24 | 3.2 |
| cis-p-t-butylcyclohexyl acetate | 4.019 | 198.31 | 5.8 |
| amyl cinnamic aldehyde | 4.324 | 202.30 | 7.3 |
| hexyl cinnamic aldehyde | 5.473 | 216.33 | 12.6 |
| cis-jasmone | 2.712 | 164.25 | 14.3 |
| geraniol | 2.649 | 154.26 | 9.8 |
| hexyl salicylate | 5.260 | 222.29 | 12.6 |

**EXAMPLE 1**

50 parts by weight of the fragrance of Example A was admixed with 50 parts by weight of NEOBEE-M5 solvent thereby forming a 'fragrance/solvent composition'. In a homogenizer as illustrated in Figures 11-A and 11-B of U.S. Patent 6,042,792, fragrance/solvent composition-containing microcapsules were prepared by interfacial polymerization of a microcapsule wall encapsulating fragrance/solvent composition droplets. To make the capsule slurry, a copolymer of acrylamide and acrylic acid was first dispersed in water together with a methylated melamine-formaldehyde pre-condensate. These two components were allowed to react under acidic conditions. The fragrance/solvent composition was then added into the solution and droplets of the desired size were achieved by high shear homogenization. Curing of the polymeric layer around the fragrance/solvent composition droplets was achieved by increasing the temperature to 50-85°C. The resulting capsule slurry contained 55% water, and 45% filled microcapsules (35% core consisting of 50% fragrance of Example A, and 50% NEOBEE M-5 and 10% microcapsule wall).

## EXAMPLE II

### FORMATION OF (a) A GEL BASE-CAPSULE SLURRY AND (b) A NON-CONFINED FRAGRANCE GEL

The ingredients set forth in the following Table IV were added together one at a time with intense agitation after each addition:

TABLE IV

| Ingredients | Parts by Weight | |
|---|---|---|
| | Capsule Slurry | Non-Confined Fragrance Gel |
| Distilled Water | 72.30 | 72.30 |
| CARBOMER 934(3% in water) | 10.00 | 10.00 |
| Microencapsulated fragrance/solvent composition prepared according to Example I | 8.60 | 0 |
| Non-Confined Fragrance of Example A | 0 | 1.51 |
| POLYOX WSR-N-3000 (3% in water) | 5.00 | 5.00 |
| 1,2-propylene glycol | 3.00 | 3.00 |
| triethanolamine | 0.40 | 0.40 |
| xanthan gum | 0.50 | 0.50 |
| methyl paraben | 0.20 | 0.20 |

The resulting slurry and the resulting non-confined fragrance-containing gel base were each then prepared for use in carrying out Examples III and IV, infra.

## EXAMPLE III

The slurry of Example II and the non-confined fragrance-containing gel base of Example II were each applied to the forearms of 10 human panelists such that one forearm on each panelist was tresated with the microcapsule-containing slurry while the other was treated with the product containing the non-confined or neat fragrance of Example A. The dosage of each product was the same. The forearms were evaluated for odor intensity by untrained judges 5, 8 and 12 hours after product application. The results are set forth in Figure 7, described supra, and have been tabulated in the following Table V:

TABLE V

| Hours | Scaled Intensity Value | |
|---|---|---|
| | Encapsulated Fragrance/solvent composition | Non-Confined Fragrance |
| 5 | 14.5 | 12 |
| 8 | 10.5 | 7.8 |
| 12 | 8.2 | 7.0 |

The results set forth in Table V clearly show that the encapsulated fragrance/solvent composition consistently provided a significantly greater at all times.

## EXAMPLE IV

The slurry of Example II and the non-confined fragrance-containing gel base of Example II were each applied to the forearms of 10 human panelists such that one forearm on each panelist was tresated with the microcapsule-containing slurry while the other was treated with the product containing the non-confined , the neat, fragrance of Example A. The dosage of each product was the same. At 8 and 12 hours after application, the forearms were rubbed with clean terry cloth towels before judging. The forearms were evaluated for odor intensity by untrained judges 5 hours

after product application and immediately after rubbing at 8 and 12 hours after application. The results are set forth in Figure 8, described supra, and have been tabulated in the following Table VI:

TABLE VI

| Hours | Scaled Intensity Value | |
|---|---|---|
| | Encapsulated Fragrance/solvent composition | Non-Confined Fragrance |
| 5 | 16 | 12 |
| 8 | 15.3 | 8.5 |
| 12 | 12.6 | 7.2 |

When comparing the above results set forth in Table VI with the results set forth in Table V of Example III, it is clear that the fragrance intensity after rubbing was significantly greater, strongly suggesting that the fragrance/solvent-containing microcapsules were being ruptured and were providing additional fragrance to the environment proximate the panelists.

**EXAMPLE V**

A moisturizing cream was prepared using the following components:

| **Component A**: | |
|---|---|
| **Ingredients** | **Parts by Weight** |
| Cyclomethicone (SILICONE 344-Dow Coming) | 10.00 |
| Shea Butter (R.LT.A.) | 2.00 |
| Lanolin Alcohol + Mineral Oil (AMERCHOL L-101) | 4.00 |
| MYRJ 59 | 0.50 |
| n-octyl methoxycinnamate | 0.50 |
| cetyl alcohol | 0.30 |
| stearic acid | 0.50 |
| propyl paraben | 0.10 |

| **Component B:** | |
|---|---|
| **Ingredients** | **Parts by Weight** |
| Distilled Water | 57.40 |
| Carbopol 934 (3% aqueous solution) | 17.00 |
| glycerine | 5.00 |
| TWEEN 60 | 0.50 |
| methyl paraben | 0.20 |

| **Component C:** | |
|---|---|
| **Ingredients** | **Parts by Weight** |
| triethanolamine | 1.00 |
| xanthan gum | 1.00 |

| Component D: | |
|---|---|
| Ingredients | Parts by Weight |
| Microencapsulated fragrance/solvent composition prepared according to Example I | 8.60 |
| Fragrance Composition of Example B | 1.50 |

The ingredients of component A were combined and heated to 75°C. The ingredients of Component B were combined and heated to 75°C. Component A was then admixed with Component B in a standard mixer until a uniform emulsion was formed. The resulting emulsion was then admixed with component C at 45°C. Component D was then admixed with the resulting mixture of components A, B and C.

3 cc. each of the resulting product was applied to a 5 square inch region of each of 10 panelist's forearms. At 8 and 12 hours after application, the forearms were rubbed with clean terry cloth towels before judging. The forearms were evaluated for odor intensity by untrained judges initially, 5 hours after product application and immediately after rubbing at 8 and 12 hours after application. Initially, the environment proximate the forearms of each of the panelists displayed an intense 'burst' of the aroma of the fragrance of Example B At 5 hours, the aroma of Example B subsided and was replaced by the lasting aroma of the fragrance of Example A. At 8 hours and at 12 hours, the forearms of each of the panelists displayed a 'burst' of the aroma of the fragrance of Example A. The results of this example indicate the advantage of including both the microencapsulated fragrance/solvent composition and a non-confined fragrance in a single skin cream whereby (a) an intitial fragrance burst' on application is achieved; (b) an ongoing fragrance evolution (in the absence of rubbing) is achieved; and (c) subsequent fragrance bursts are achieved as a result of rubbing which ruptures a significant portion of the microcapsules applied when the slurry was applied.

Each of the specifications and claims of the U.S. Patents, Patent Applications and Published U.S. Patent Applications is herein incorporated by reference as if set forth in their entirety.

## Claims

**1.** A human epidermal and/or hair treatment composition comprising an admixture of (i) a plurality of microcapsules each of which (a) has an outside diameter in the range of from about 0.01 to about 1000 microns; (b) has a wall having a thickness in the range of from about 0.001 to about 100 microns and has a core comprising a liquid selected from the group of fragrances, solvents, malodor counteractant compositions, beneficial agents and mixtures of fragrance, solvent, malodor counteractant compositions and beneficial agents, wherein greater than about 50 weight percent of the liquid has a C $\log_{10}P$ of from about 1.5 to about 8.0; and wherein the solvent composition (A) is compatible with said fragrance composition and/or said malodour counteractant composition and (B) has a C $\log_{10}P$ greater than about 2.0 wherein P represents the n-octanol/water partition coefficient for each of said fragrance composition components and/or said malodour counteractant composition components and P' represents the n-octanol/water partition coefficient for each of said solvent components and (ii) a human epidermal skin treatment and/or hair treatment base for maintaining in suspension said plurality of microcapsules, selected from the group consisting of a gel base, a suspension, a powder and an emulsion base, the concentration of fragrance composition components and/or malodour counteractant composition components in said human epidermal and/or hair treatment composition being in the range of from about 0.1 to about 50.0% by weight of said human epidermal and/or hair treatment composition.

**2.** A human epidermal compostion of claim 1 wherein the range of weight percent of fragrance composition components and/or malodour counteractant composition components in said microcapsules being from about 5 % to about 98 % by weight of filled microcapsules; and the range of weight percent of solvent in said plurality of microcapsules being from about 10% to about 90% by weight of said microcapsules.

**3.** A human epidermal and/or hair treatment composition of claim 1 comprising an admixture (i) of microcapsules having (a) an outside diameter in the range of from about 0.01 to about 1000 microns; (b) a wall having a thickness in the range of from about 0.001 to about 100 microns; (c) a wall composed of a substituted or un-substituted acrylamide-acrylic acid co-polymer cross-linked with a melamine-formaldehyde and/or a urea-formaldehyde precondensate; and (d) has a liquid phase core selected from the group consisting of a fragrance composition, a malodour counteractant composition, a beneficial agent and mixtures of the components of which has a C $\log_{10}P$ of from about 2.0 to about 8.0 and a solvent composition of the components of which (A) is compatible with the

components of said fragrance composition, said malodour counteractant or beneficial agent composition and (B) has a C $\log_{10}P$ greater than about 2.0 wherein P represents the n-octanol/water partition coefficient for said fragrance composition components and/or said malodour counteractant composition components and P represents the n-octanol/water partition coefficient for each of said solvent components and (ii) a human epidermal skin treatment and/or hair treatment base for maintaining in suspension said plurality of microcapsules, selected from the group consisting of a gel suspension, powder and an emulsion base, the concentration of fragrance composition components and/or malodour counteractant composition components in said human epidermal and/or hair treatment composition being in the range of from about 0.1 to about 50.0% by weight of said human epidermal and/or hair treatment composition; the range of weight percent of fragrance composition components and/or malodour counteractant composition components in said plurality of microcapsules being from about 5% to about 98% by weight of filled microcapsules; and the range of weight percent of solvent in said plurality of microcapsules being from about 10% to about 90% by weight of filled microcapsules.

**4.** The human epidermal and/or hair treatment composition of claim 1 wherein each of the microcapsules has an average diameter is the range of from about 0.05 microns to about 100 microns and an average wall thickness in the range of from about 0.005 microns to about 10 microns.

**5.** The human epidermal and/or hair treatment composition of claim 1 wherein each of the rupturable microcapsules has an average diameter is the range of from about 2.0 microns to about 15 microns and an average wall thickness in the range of from about 0.2 microns to about 2.0 microns.

**6.** The human epidermal and/or hair treatment composition of claim 1 wherein the components of the hydrophobic solvent composition has a C $\log_{10}P$ greater than about 8.

**7.** The human epidermal and/or hair treatment composition of claim 1 wherein the solvent is selected from the group consisting of a mono-, di- or tri-$C_4$-$C_{26}$ saturated or unsaturated fatty acid glyceride, diethyl phthalate, dibutyl phthalate, diisodecyl adipate, a liquid polydimethyl siloxane, a liquid polydimethylcyclosiloxane, the methyl ester of soya fatty acid, a mixture of soya fatty acid methyl ester and isopropyl myristate with the weight ratio of soya fatty acid:isopropyl myristate being from 2:1 to 20:1 and a mineral oil compatible with each component of said fragrance composition and/or said malodour counteractant composition.

**8.** The human epidermal and/or hair treatment composition of claim 7 wherein the hydrophobic solvent is the triglyceride ester of a mixture of caprylic acid and capric acid.

**9.** The human epidermal and/or hair treatment composition of claim 1 wherein at least a fraction of said plurality of microcapsules are coated with a cationically-charged polymer and/or a non-ionic polymer.

**10.** The human epidermal and/or hair treatment composition of claim 1 wherein at least a fraction of said plurality of microcapsules, the walls of which are cured at a temperature of from 50°C to about 85°C.

**11.** The human epidermal and/or hair treatment composition of claim 1 wherein the average outside diameter of each of the microcapsules is from about 0.05 microns to about 100 microns.

**12.** The human epidermal and/or hair treatment composition of claim 1 wherein the aqueous human epidermal skin and/or hair treatment base is a gel base having a pH in the range of from about 4.5 to about 9, comprising water, at least one suspending agent, at least one gelling agent, at least one pH buffering agent and at least one moisturizer.

**13.** The human epidermal and/or hair treatment composition of claim 1 wherein the aqueous human epidermal skin and/or hair treatment base is an emulsion base having a pH in the range of from about 4.5 to about 9, comprising water, at least one suspending agent, at least one pH buffering agent, at least one emulsifier, at least one emulsion stabilizer and at least one moisturizer.

**14.** The human epidermal and/or hair treatment composition of claim 12 wherein the suspending agent is a solid suspending agent selected from the group consisting of xanthan gum, guar gum, attapulgite clay, hydroxypropyl cellulose having a molecular weight of from about 50,000 to about 800,000, colloidal silica, and ethyl cellulose having a particle size of from about 0.004 to about 0.130 microns, a surface area of from about 100 to about 500 $m^2$ per gram and a density of from about 1.0 to about 4.0 pounds per cubic foot.

**15.** The human epidermal and/or hair treatment composition of claim 13 wherein the suspending agent is a solid suspending agent selected from the group consisting of xanthan gum, guar gum, attapulgite clay, hydroxypropyl cellulose having a molecular weight of from about 50,000 to about 800,000, colloidal silica, and ethyl cellulose having a particle size of from about 0.004 to about 0.130 microns, a surface area of from about 100 to about 500 $m^2$ per gram and a density of from about 1.0 to about 4.0 pounds per cubic foot.

**16.** The human epidermal and/or hair treatment composition of claim 12 wherein the gelling agent is a polymer of acrylic acid cross-linked with an allyl ether of sucrose.

**17.** A process for treating a given region of the human epidermis and/or human hair comprising the step of applying to said given region of the human epidermis and/or hair a human epidermis and/or hair treating quantity of the composition of claim 1 over a human epidermis and/or hair treatment period of time.

**18.** The process of claim 16 wherein the intensity of fragrance evolved from the microcapsules applied to the human epidermis or hair follicles as a result of the application of the human epidermal and/or hair treatment composition, as a function of time, is in accordance with the algorithm:

$$Y = A \operatorname{Log}_e X + B$$

wherein Y is a scaled measure of fragrance intensity on a scale of 0 - 20 and X is time as measured in hours subsequent to the application of the human epidermal and/or hair treatment composition; and wherein A is in the range of from -3.0 to -8.0 and B is in the range of from 22 to 26.

**19.** The human epidermal and/or hair treatment composition of claim 1 wherein the plurality of microcapsules has a wall composed of an unsubstituted acrylamide-acrylic acid copolymer having a molecular weight in the range of from 5,000 to 1,000,000 cross-linked with a melamine-formaldehyde pre-condensate, wherein the mole ratio of acrylic acid monomeric units:acrylamide monomeric units is from 9:1 to 1:9 and wherein the mole ratio of melamine-formaldehyde precondensate cross-linking agent:acrylamide-acrylic acid copolymer is in the range of from 9:1 to 1:9.

**20.** The human epidermal and/or hair treatment composition of claim 19 wherein the mole ratio of acrylic acid monomeric units:acrylamide monomeric units is from 7:3 to 3:7.

**21.** The human epidermal and/or hair treatment composition of claim 19 wherein the mole ratio of melamine-formaldehyde precondensate cross-linking agent:acrylamide-acrylic acid copolymer is in the range of from 5:1 to 1:5.

**22.** The human epidermal and/or hair treatment composition of claim 21 wherein the mole ratio of melamine-formaldehyde precondensate cross-linking agent:acrylamide-acrylic acid copolymer is in the range of from 2:1 to 1:2.

**23.** The human epidermal and/or hair treatment composition of claim 19 wherein the unsubstituted acrylamide-acrylic acid copolymer has a molecular weight in the range of from 10,000 to 100,000.

**24.** The human epidermal and/or hair treatment composition of claim 1 wherein the aqueous human epidermal skin and/or hair treatment base comprises ethanol, at least one suspending agent and water.

**25.** A method for treating a given region of the human epidermis and/or hair comprising the step of applying to said given region of the human epidermis and/or hair a human epidermis and/or human hair treating quantity of the composition of claim 24 over a human epidermis and/or human hair treatment period of time.

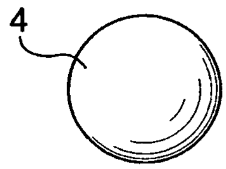


FIG. 1

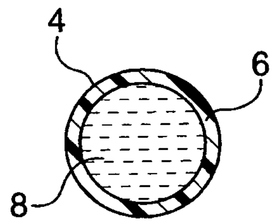


FIG. 2

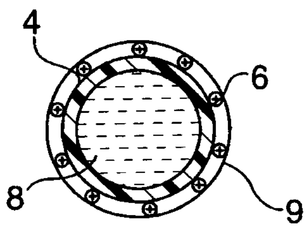


FIG. 3

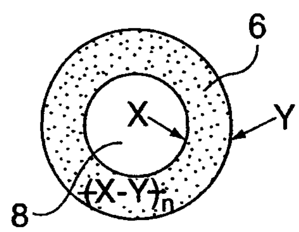


FIG. 4A

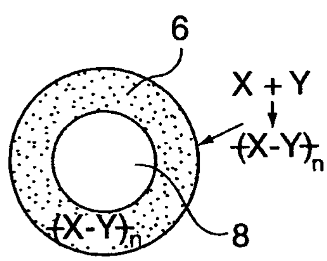


FIG. 4B

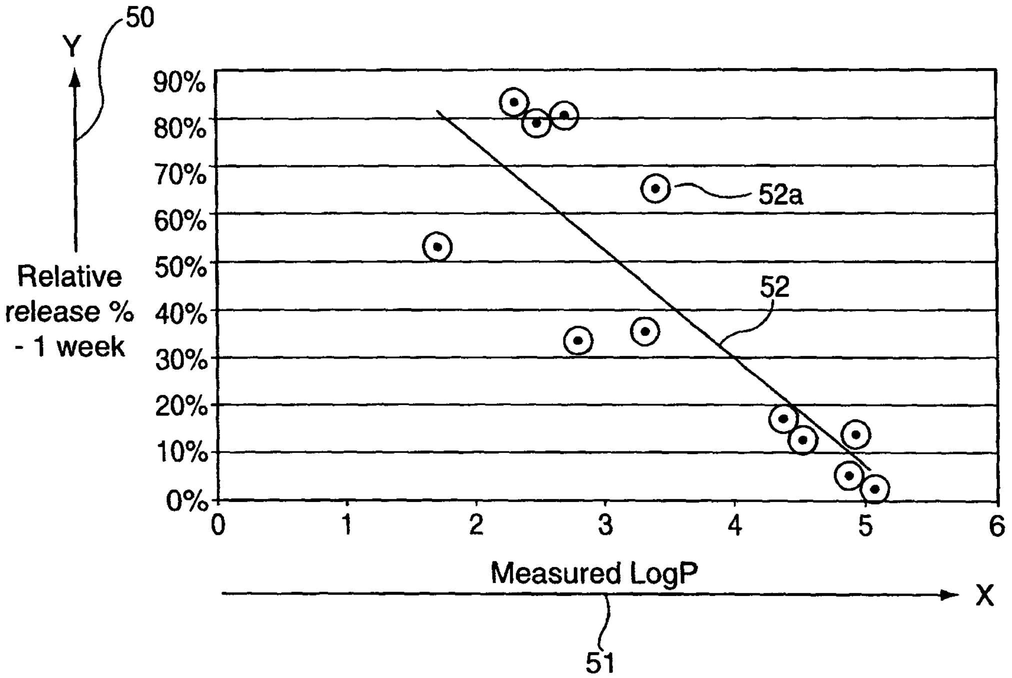
50
Y
Relative
release %
- 1 week
90%
80%
70%
60%
50%
40%
30%
20%
10%
0%
0
1
2
3
4
5
6
52a
52
Measured LogP
X
51

FIG. 5

FIG. 6

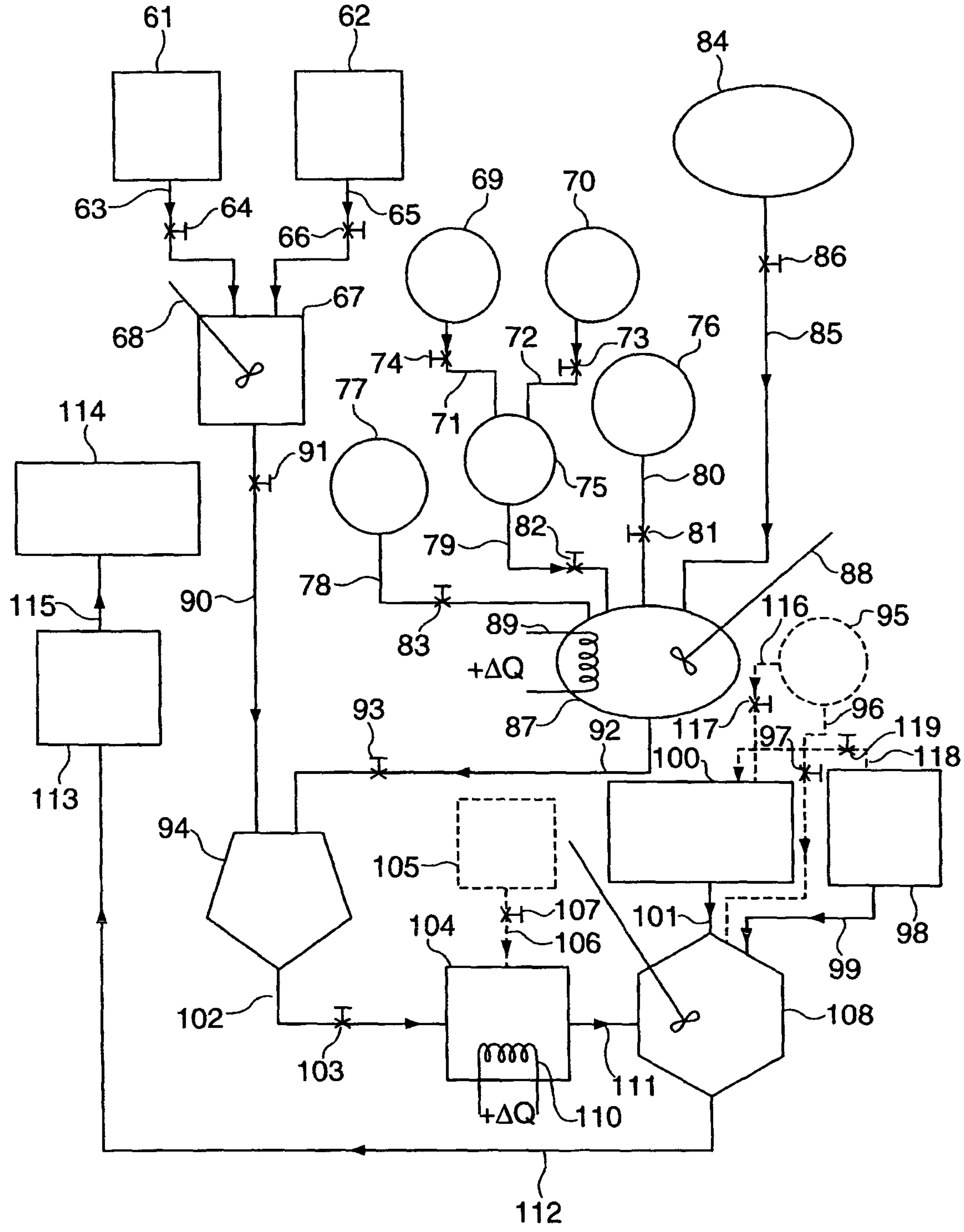
61
62
84
63
64
65
66
69
70
86
85
67
68
74
72
73
76
71
77
75
114
91
80
79
82
81
88
90
78
83
115
89
116
95
+ΔQ
87
117
96
93
92
97
119
118
100
113
94
105
107
101
98
104
106
99
102
103
108
111
110
+ΔQ
112

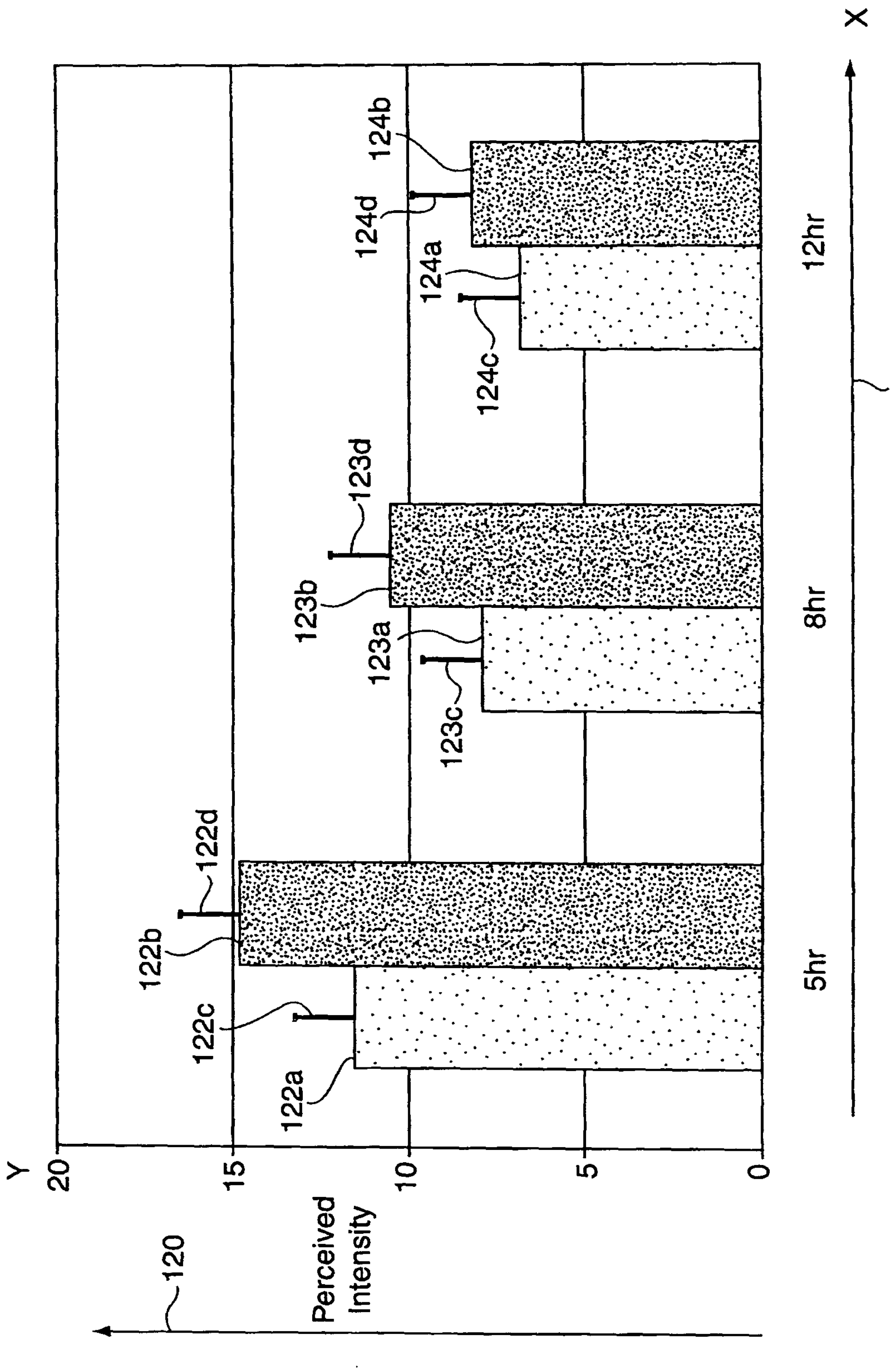

Y
20
15
10
5
0
Perceived Intensity
120
X
121
5hr
8hr
12hr
122a
122b
122c
122d
123a
123b
123c
123d
124a
124b
124c
124d


FIG. 7

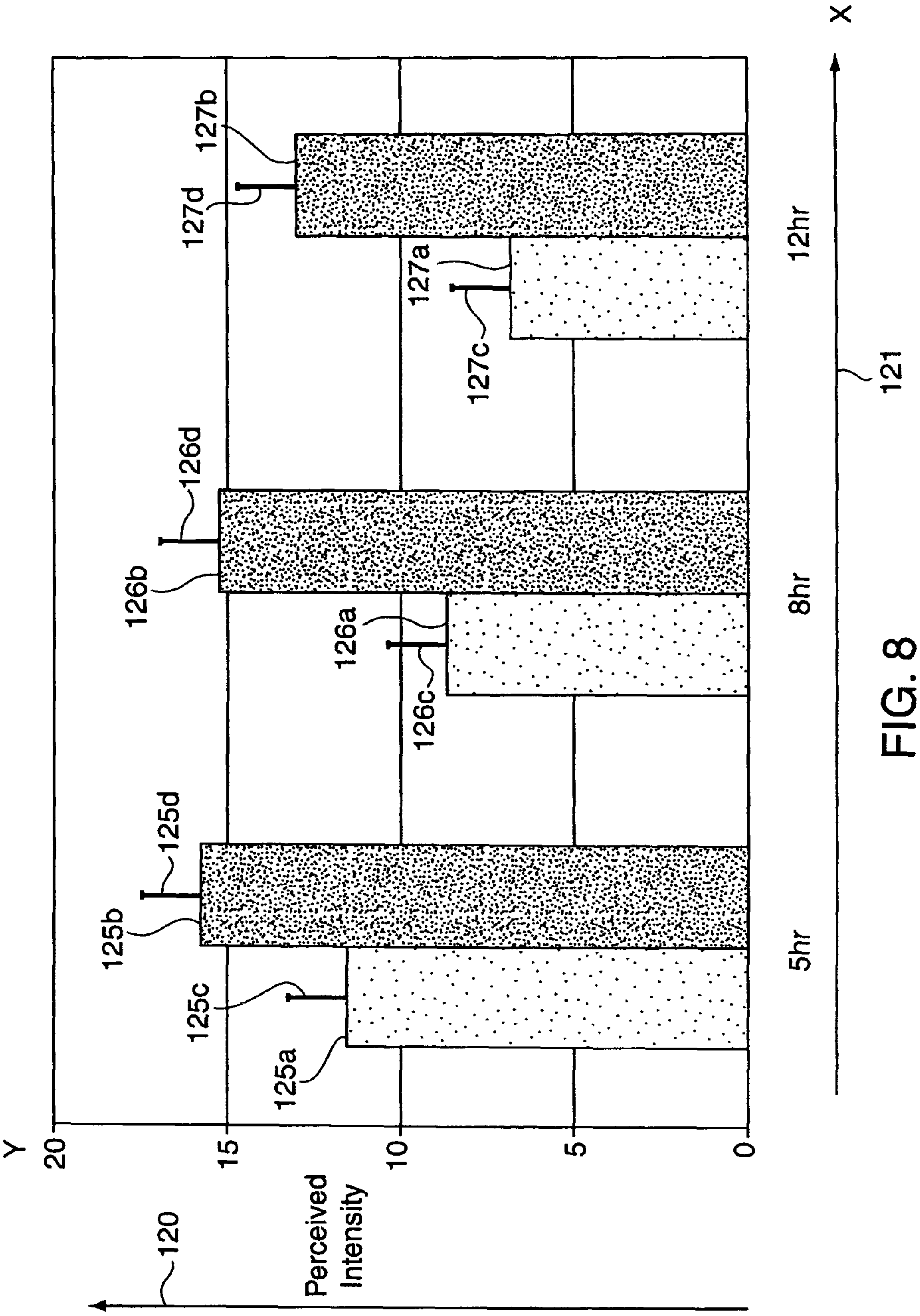
Y
20
15
10
5
0
Perceived Intensity
120
X
121
5hr
8hr
12hr
125a
125b
125c
125d
126a
126b
126c
126d
127a
127b
127c
127d

FIG. 8

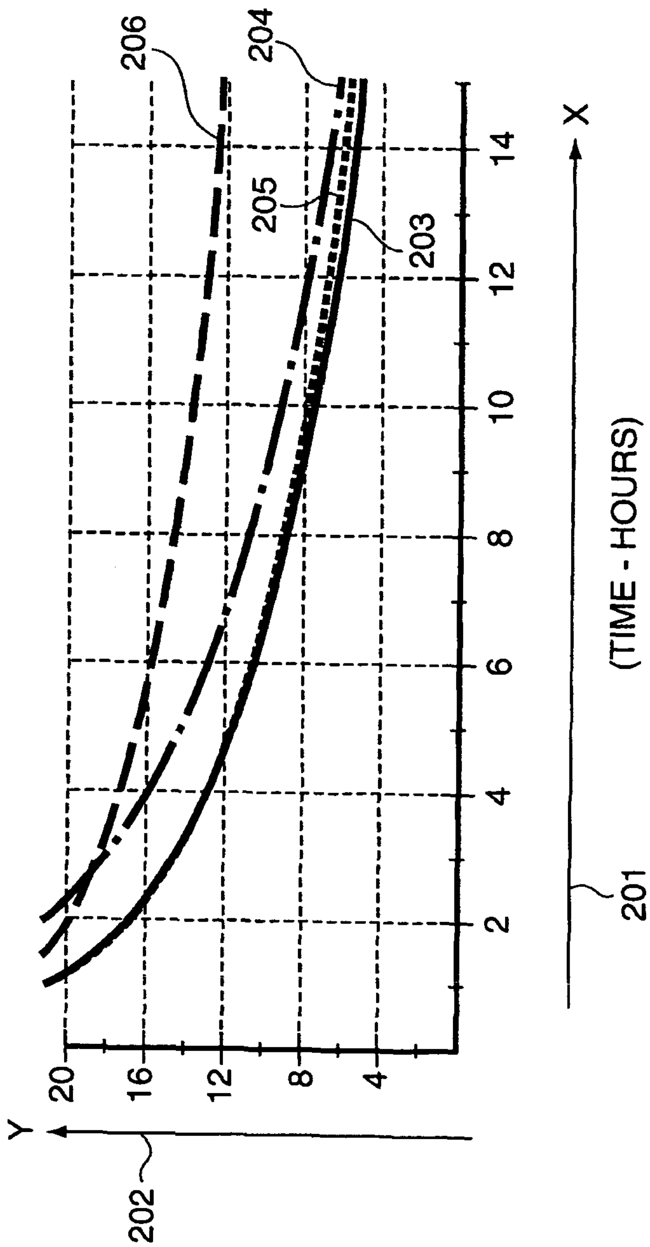


FIG. 9

European Patent Office

# EUROPEAN SEARCH REPORT

Application Number
EP 05 25 2299

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 2004/016234 A (QUEST INTERNATIONAL B.V) 26 February 2004 (2004-02-26)<br>* page 7, lines 5-20; examples *<br>* page 9, last paragraph - page 10, paragraph 1 *<br>* page 2, lines 5-30 * | 1-25 | B01J13/00<br>A61K7/50<br>A61K7/16<br>A61K7/46 |
| X | US 6 050 129 A (SHEFER ET AL) 18 April 2000 (2000-04-18)<br>* figures 17,18a; examples A,III * | 1-25 | |
| X | EP 0 965 326 A (THE PROCTER & GAMBLE COMPANY) 22 December 1999 (1999-12-22)<br>* paragraphs [0048], [0049], [0136]; examples 1-3 * | 1-25 | |
| X | WO 01/62376 A (HENKEL KOMMANDITGESELLSCHAFT AUF AKTIEN) 30 August 2001 (2001-08-30)<br>* example 2 * | 1-25 | |
| X | EP 0 316 054 A (SHISEIDO COMPANY LIMITED) 17 May 1989 (1989-05-17)<br>* examples * | 1-25 | TECHNICAL FIELDS SEARCHED (Int.Cl.7)<br>B01J<br>A61K |
| E | WO 2005/055964 A (SALVONA LLC) 23 June 2005 (2005-06-23)<br>* figures 1,2; examples * | 1-25 | |

The present search report has been drawn up for all claims

1

| Place of search | Date of completion of the search | Examiner |
|---|---|---|
| Munich | 21 July 2005 | Miller, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 05 25 2299

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2005

| Patent document cited in search report | Publication date | Patent family member(s) | Publication date |
|---|---|---|---|
| WO 2004016234 A | 26-02-2004 | EP 1393706 A1 | 03-03-2004 |
| | | AU 2003251377 A1 | 03-03-2004 |
| | | BR 0305777 A | 05-10-2004 |
| | | EP 1534216 A1 | 01-06-2005 |
| | | WO 2004016234 A1 | 26-02-2004 |
| US 6050129 A | 18-04-2000 | CA 2246639 A1 | 18-03-1999 |
| | | DE 908174 T1 | 17-02-2000 |
| | | EP 0908174 A2 | 14-04-1999 |
| | | ES 2133260 T1 | 16-09-1999 |
| | | ID 22071 A | 02-09-1999 |
| | | JP 11171754 A | 29-06-1999 |
| | | SG 75872 A1 | 24-10-2000 |
| EP 0965326 A | 22-12-1999 | EP 0965326 A1 | 22-12-1999 |
| | | AU 3949999 A | 05-01-2000 |
| | | BR 9911218 A | 06-03-2001 |
| | | CA 2334752 A1 | 23-12-1999 |
| | | CN 1305367 A ,C | 25-07-2001 |
| | | WO 9965458 A1 | 23-12-1999 |
| | | JP 2002518525 T | 25-06-2002 |
| | | MA 24878 A1 | 31-12-1999 |
| | | US 6869923 B1 | 22-03-2005 |
| WO 0162376 A | 30-08-2001 | DE 10008306 A1 | 06-09-2001 |
| | | DE 10008307 A1 | 06-09-2001 |
| | | DE 10008305 A1 | 06-09-2001 |
| | | AT 281235 T | 15-11-2004 |
| | | AU 4645901 A | 03-09-2001 |
| | | DE 50104385 D1 | 09-12-2004 |
| | | WO 0162376 A1 | 30-08-2001 |
| | | EP 1257353 A1 | 20-11-2002 |
| | | ES 2231467 T3 | 16-05-2005 |
| EP 0316054 A | 17-05-1989 | JP 1238513 A | 22-09-1989 |
| | | JP 2639816 B2 | 13-08-1997 |
| | | JP 1265007 A | 23-10-1989 |
| | | JP 1266846 A | 24-10-1989 |
| | | JP 2700069 B2 | 19-01-1998 |
| | | JP 1268620 A | 26-10-1989 |
| | | JP 1268621 A | 26-10-1989 |
| | | JP 1268622 A | 26-10-1989 |
| | | JP 1125313 A | 17-05-1989 |
| | | JP 2097182 C | 02-10-1996 |
| | | JP 7121850 B | 25-12-1995 |
| | | DE 3882906 D1 | 09-09-1993 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 25 2299

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2005

| Patent document cited in search report | Publication date | Patent family member(s) | Publication date |
|---|---|---|---|
| EP 0316054 A | | DE 3882906 T2 | 23-12-1993 |
| | | EP 0316054 A1 | 17-05-1989 |
| | | US 5089269 A | 18-02-1992 |
| | | US 6231873 B1 | 15-05-2001 |
| WO 2005055964 A | 23-06-2005 | US 2004175404 A1 | 09-09-2004 |
| | | WO 2005055964 A1 | 23-06-2005 |

EPO FORM P0459

For more details about this annex : see Official Journal of the European Patent Office, No. 12/82